(19)

Europäisches
Patentamt

European
Patent Office

Office européen
des brevets

(11)  **EP 4 549 575 A1**

(12)  **EUROPEAN PATENT APPLICATION**

(43) Date of publication:
**07.05.2025   Bulletin 2025/19**

(21) Application number: **23207559.8**

(22) Date of filing: **02.11.2023**

(51) International Patent Classification (IPC):
***C12N 15/82*** *(2006.01)*    ***A01N 63/50*** *(2020.01)*

(52) Cooperative Patent Classification (CPC):
**C12N 15/8282; A01N 43/713; A01P 3/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Universita' degli Studi di Milano
20122 Milano (IT)**
• **KTH Holding Aktiebolag
100 44 Stockholm (SE)**
• **Université Paris-Saclay
91190 Gif-sur-Yvette (FR)**

(72) Inventors:
• **Pesaresi, Paolo
Milan (IT)**
• **Masiero, Simona
Milan (IT)**
• **Rosa, Stefano
Milan (IT)**
• **Mizzotti, Chiara
Milan (IT)**
• **Tagliani, Andrea
Milan (IT)**

• **Pellegrino, Sara
Milan (IT)**
• **Feni, Lucia
Milan (IT)**
• **Toffolatti, Silvia
Milan (IT)**
• **Marcianò, Demetrio
Milan (IT)**
• **Bulone, Vincent
Stockholm (SE)**
• **Srivastava, Vaibhav
Stockholm (SE)**
• **Ongeri, Sandrine
Gif sur Yvette (FR)**
• **Radal, Léa
Gif sur Yvette (FR)**

(74) Representative: **Calogero, Ida et al
BIRD & BIRD SOCIETA TRA AVVOCATI S.R.L.
Via Porlezza, 12
20123 Milano (IT)**

Remarks:
The complete document including Reference
Table(s) and the Sequence Listing(s) can be
downloaded from the EPO website

(54)  **ANTIMICROBIAL PEPTIDES FOR THE CONTAINMENT OF OOMYCETES IN AGRICULTURE**

(57)    The present invention relates to novel antimicrobial peptides for use as a viable and sustainable alternative to fungicides currently used in agriculture against infection by oomycetes infection, and related methods for protecting crops.

**EP 4 549 575 A1**

### *Phytophthora infestans* Cellulose synthase 3: protein sequence

```
MGLTGAGIIASVVGILGGVSLSCGGWSSLSLGARSLFVTTQFLSAFAMGFVVAFSAIVSLSDTNEW
VAVAAGGGAGFVIALIGGFMTIFGPYILILITGGLIACYLLLVDAYDGINVFPADNQLARQEFVIA
FMIIFELVCCSSSKTSELENHRFKYIVFSAITGGWMAADGVSRLIDSGAVLSTVAYTSIQDGGKAA
MDGIDASAQTLMFVIWGAVFVVGGLNQLSMRWGLMCYNRVGAHAQLGPVEEQMPELPTGATLPAQT
MTERVRLVCENCFATVPAGTAFCTECGEAMPSEDGNPDVSISQAQMPSVSMNNKGQVPDRWQHVPH
RTYMSTTSFVDPKHAKEGGVSMKDNGRSIRFMDSGVQGPDGKMSQYNDSIAGVRNYYEPSFRSFAM
STYSIANRAAEPVETPNIRKYKMSGSGMFHVFYFGTAATGIFWLYYLTTMYPQQYFCDHARPTLPC
SGLPTSETTGCYSSTVNFDADSGDGYCIKDVPFMSWLMYAMMIFSEFLNYFLGLLFNFSMWRPIRR
GARYMNDFKPPIPKEQWPTVDIFLCHYMEPVTDSMQTLKNCLAMQYPPELLHIFILDDGYTKSVWD
ANNHFKVTVNTKVIEVAGDLRGDLARLMHERVVGPVQDDQSLKSWRRQHSSVRELRKEGGKGVQRR
DCAVGSLSDDYDYRDRGIPRVTFIGRMKPETHHSKAGNINNALFNEGADGKYLLILDNDMKPHPKF
LLAVLPFFFSEGEAVDGGGRQYSDDISWNQVSYVQTPQYFEDTPQLTIMGDPCGHKNTIFFDAVQC
GRDGFDSAAFAGTNAVFRRQAFDSIGGICYGTQTEDAYTGNVLHTSGWDSVYFRKDFEGDAKDRIR
LCEGAVPDTVAAAMGQKKRWAKGAVQILLMKNESEVDPDWRPPRVPAPDPKPSLTFPRKMFFYDSV
LYPLGSIPALCYVSIAVYYLCTGDAPIYARGTKFLYSFLPVTFCRWVLNLLANRAVDNNDVWRAQQ
TWFSFSFITMMAIVEAIQARVTGKDKSWANTGAGQKTSWTEIPNVLFFFTLLFSQLVALVRFFEYE
NATNPWNYVSAMFFGFFVMSQFYPMVKMSITEYCGWDHTAATFTANVFGSLLVVYIVVFVQLWQVY
YEGNLQVAQGTDAGGSAAAT*
```

### *Phytophthora infestans* Cellulose synthase 2: protein sequence

```
MYGNDKQSLMKHEDYELHGTPATGANDGGAGFYAQEGRPTPQQGYVDPRGPALPPMNVSDAVGLGG
QRDNIISVHGYMHKQGKRTIKGPIHKSWKRRYFALEKAKIYYFYSHLECRQYFTTRNADLVVGAIE
LKDALQLRPCARLDLPHKGFEVHTKRRVWVLCPETDEEYRMWFQGVERAIVANGAGNIIERKLPNV
RKYLMKGNQTYRFFYFLFLIAGIVELLAIVFWFVIGLEPCDASRLEVDCATITSTSLETLRCSNEP
FSGWFTPPDWYLEVADVDNVICFRDPPIPQWASYFAMLFAEVLTFALGVLYYLGMWKPVRRGAHYF
DEFEPFVPDDLWPKVDVLLCHYSEPAEETIDTLMACMNLQYPPHLLQIWVCDDGYCKTKWTKGNPV
PTVELNKGILETAGDLRQEVAQFMYDRVCDPNEDMEVYAWRKLHSSANLPSPSRVKVVNRADCAVG
SFRDDYRYPGLPHVTFIGRVKPETHYSKAGNINNCLYNEGANGRYLIILDTDMQPHPKFILATLPF
FFDDEDRQDKAKYICCGIGCNAVAKLCCASCQIAGVPEEQISYCSKDCFENAMHVQSAVHRRQVNG
TMSDTRASKIDMRCMNCDAKLPKNGVCRKCGNHGADGEDVSSLHNYSDDVRDNAVAFVQTPQYFRD
CIQLQIGDPMGHRNATFYDAIQTGQDGYDCASFAGTNAMFRREALDSIGGIQYGSLTEDCYTGQVL
CSMGWKAQYFRKDFEGEPSERIRLAEGLIPDSVAGSLAQRKRWAKGNFQIALMNKKTQYFDPEWKM
PEAQIPSYHKSNKFMRRVFYFNSTLYPLGSITAILFYYITLYFLFSGYAPIYMAGARLVYALVPKL
LIQGVLSALSNRTVDNSDVIRSQEVWFAYAFTNCTAVLEAFWWKITGKEPKWFNTGGASRGSTAEL
PNVIIFFGTVVGVLWSVVRFLAGYNSIQTSHGASLLFASLMMGLFIAVKLAPSVRMSIQEYFGWSY
ESLMDQGNVVGSISIAFGLIFITLWVWIEQPTSNPF*
```

**FIG. 1**

## Description

The present invention relates to novel antimicrobial peptides for use as a viable and sustainable alternative to fungicides currently used in agriculture against oomycete infections, and related methods for protecting crops.

## BACKGROUND

Oomycetes are filamentous eukaryotic organisms belonging to the TSAR supergroup, of which around 1200 species are known, many of which pose a threat to agricultural activities [1]. Oomycetes, together with fungi, are the aetiological agents of important plant diseases and thus cause major crop losses. Losses are recorded between 10 and 23 % in the pre-harvest phase, to which further damage is added in the storage phase, where estimates indicate losses of around 10-20 % [2].

Among the most important phytopathogenic oomycetes are:

1) *Phytophthora infestans* Mont. (de Bary): is one of the most destructive phytopathogenic oomycetes. This organism is the aetiological agent of potato and tomato blight, and is sometimes harmful to other plants belonging to the Solanaceae family. *P. infestans* is responsible for huge crop losses each year with a global cost conservatively estimated at over $6 billion per year [3].

2) *Phytophthora capsici* Leonian: present in North and South America, Asia, Africa and Europe [4], it is responsible for root, crown, leaf and fruit rots. This pathogen is capable of infecting more than 26 plant families, including Cucurbitaceae, Solanaceae, and Fabaceae [5].

3) *Pythium ultimum* Trow var. ultimum: is a ubiquitous pathogen, the aetiological agent of root rot and seedling wilt and seed-bed mortality in numerous crop species (around 300 different hosts), including major species such as maize and wheat [6].

4) *Plasmopara viticola* Berl. & De Toni: is the causal agent of grapevine downy mildew, one of the most widespread and destructive diseases affecting species of the genus Vitis [7]. The most common symptoms of the disease include necrosis of the stems, wilting of the berries and chlorosis of the leaves. Severe attacks usually lead to premature leaf fall (phylloptosis). In the absence of treatment and under favorable weather conditions, downy mildew can destroy up to 75% of the crop in a single season and can weaken new shoots, causing loss of vigour and reduced production even in subsequent years, resulting in heavy production losses [8].

Currently, defense against these pathogens is carried out through the frequent application of fungicides, both of natural origin (such as copper) and consisting of synthetic active ingredients. Although the European Food Safety Authority stated that 97% of agricultural products analyzed were below the legal limits for residues of phytosanitary substances (EFSA reports, 2013), several foods, including table grapes and wine, are considered to be at medium to high risk for fungicide residue content. Furthermore, the regular use of fungicides may pose a potential risk to the environment, due to the persistence of residues in the soil and possible contamination of watercourses and/or groundwater [9]. The use of copper, for example, raises concerns because it can damage soil organism populations (earthworms and various microorganisms), and also poses a risk to soil fertility in the long term [10]. Also, to be taken into account is the fact that some active ingredients have poor degradability and therefore remain in the environment for a long time.

In addition to the environment, continuous exposure to fungicides can also have negative consequences for human health. Direct contact with the substances (through skin, eyes, ingestion or inhalation) can cause variable reactions, ranging from mild to severe (acute toxicity). Moreover, due to their widespread use in agriculture, the population is subjected to continuous exposure to low doses of fungicides through the environment and food (chronic toxicity). The possible human health consequences of such continuous exposure are beginning to be clarified. The possibility that environmental pollutants may interfere with the human immune system has been raised by an increasing number of studies [11-12].

In addition, plant protection products entering the food chain may encounter the phenomenon of biomagnification, i.e., the tendency of certain chemicals to become increasingly concentrated as they move up the trophic chains. As a result, accumulated concentrations in the tissues of organisms can be many times higher than in the surrounding environment.

It should also be added that the massive and repeated use of single-target fungicides in combination with intensive agriculture, characterized by large, genetically uniform crops, favors the emergence of new virulent strains; it therefore becomes necessary to develop alternative strategies to achieve greater sustainability while ensuring crop continuity. This is a partial list of the negative effects that agricultural technologies and practices can have on the ecosystem, and which justify the need to develop new antimicrobial compounds that can be used in agriculture to control plant diseases, characterized by low toxicity and reduced environmental impact (see also Directive 2009/128/EC).

The authors of the present invention have now identified innovative synthetic antifungal cyclic peptides and their linear versions capable of inhibiting the growth of *P. infestans, P. capsici, P. ultimum* and *P. viticola.* These are peptides with improved activity, specificity, biodegradability and toxicity properties compared to conventional plant protection products.

These peptides may be advantageously used in integrated management programs for agronomically relevant pathogens or as an alternative to traditional copper-based fungicides, enabling a major reduction in their use as required by the European Commission's 'Farm to Fork' (F2F) program (https://food.ec.europa.eu/horizontal-topics/farm-fork-stra tegy_en), which envisages a 50% reduction in chemical plant protection products by 2030.

Thus, it is an object of the present invention a linear or a cyclic peptide consisting of a β-amino acid and 8 L-amino acids having the following sequence (I):

X - R - (Y1-Y2-Y3-Y4-Y5-Y6) - Z          (I)

wherein X is selected from the group consisting of β-Ala (βA), Ala (A), Gly (G), gamma amino butyric acid or a derivative thereof;

R is an L-amino acid selected between Arg (R) or Gly (G);
Y1, Y2, Y3, Y4, Y5 and Y6 are L-amino acid selected from the group consisting of Trp (W), Leu (L), Met (M), Tyr (Y), Arg (R) or Val (V);
Z is an L-amino acid selected between Arg (R) or Val (V);
or their peptidomimetic variants;
with the proviso that the amino acid Arg (R) is present at least three times and Trp (W), Met (M), Tyr (Y), Ala (A), Gly (G) and Val (V) are present once in the sequence of formula (I), as antimicrobial or fungicide agent in agronomic field.

In a preferred embodiment of the invention the meaning of amino acid residues $Y_1$-$Y_6$ in above formula (I) is the following:

$Y_1$ is Trp (W) or Arg (R);
$Y_2$ is Met (M) or Leu (L);
$Y_3$ is Leu (L) or Arg (R);
$Y_4$ is Val (V) or Trp (W);
$Y_5$ is Tyr (Y) or Leu (L) and
$Y_6$ is Arg.

According to the present invention with the expression a derivative of β-Ala, Ala, Gly or gamma amino butyric acid (i.e. the X meaning) it is intended that the amino acid which is or not substituted on alpha, beta or gamma position by an alkylCO group wherein alkyl is a linear or branched alkyl group $C_1$-$C_4$. Preferably, said linear alkyl group may be selected among acetyl, ethyl, propyl, butyl group.

In a preferred embodiment of the invention X is a derivative of β-Ala, and preferably is acetyl-βA (Ac-βA).

In a most preferred embodiment of the invention the cyclic peptides are selected between the following sequences:

βA-R-W-M-L-V-Y-R-R (SEQ ID NO:1) (indicated also cyclic peptide CP20, after head-tailed cyclization from linear peptide LP20)
βA-G-R-L-R-W-L-R-V (SEQ ID NO:2) (indicated also as cyclic peptide CP32 after head-tailed cyclization from linear peptide LP32)

and the linear peptides are selected between the following sequences:

Ac-βA -R-W-M-L-V-Y-R-R-$CONH_2$ (SEQ ID NO:3) (indicated also as linear peptide Ac-LP20)
Ac-βA -G-R-L-R-W-L-R-V-$CONH_2$ (SEQ ID NO:4) (indicated also as linear peptide Ac-LP32).

It is also included in the scope of the present invention a variant of the linear amino acid sequences of the peptides wherein the carboxylic group of the C-term amino acid is substituted by an amide group $CONH_2$ or by a CONHalkyl group or is esterified with a linear or branched $C_1$-$C_4$ alkyl group. Preferably, said alkyl or said linear $C_1$-$C_4$ alkyl group is selected from the group consisting of methyl, ethyl, propyl, butyl group.

The linear peptide sequences SEQ ID NO:3 and SEQ ID NO:4 according to the invention are examples of such derivatization wherein a C-terminal amide group is present as indicated in the formula by the addition of "$CONH_2$".

The present invention also contemplates the peptidomimetic variants of said peptides, i.e. molecules that, while maintaining the structure and the bioactivity of the original molecule, exhibit greater stability (given by better resistance to proteolysis and/or to both physical and chemical degradation). These variants derive from the incorporation of not proteinogenic amino acids, such as D-amino acids, N-alkyl glycines, fluorinated amino acids, aza amino acids, hydrazino acids, or beta-amino acids.

By way of example, peptide fluorination is an effective strategy to improve the stability of the peptides of the invention with respect to enzymatic, chemical and thermal denaturation. Fluoroalkyl groups increase local hydrophobicity and facilitate membrane traversing.

In a preferred embodiment of the invention the peptidomimetic variants of linear or cyclic peptide having SEQ ID NO:1 or SEQ ID NO:3 are selected from the group consisting of:

βA-R-W-M-aL-V-Y-R-R (SEQ ID NO:7)
βA-R-W-M-hL-V-Y-R-R (SEQ ID NO:8)
βA-R-W-MCF3-L-V-Y-R-R (SEQ ID NO:9)
βA-(D)R-(D)W-M-L-V-(D)Y-R-(D)R (SEQ ID NO:10)
βA-(D)R-(D)W-M-L-V-(D)Y-R-R (SEQ ID NO:11)
βA-R-(D)W-M-L-V-(D)Y-R-R (SEQ ID NO:12)
βA-R-W-A-hL-V-Y-R-R (SEQ ID NO:13)
βA-R-(D)W-M-L-V-(D)Y-R-R (SEQ ID NO: 14)
wherein aL is aza-Leu; hL is hydrazino-Leu;; MCF3 is CF$_3$-Met and (D) indicates that it is a D-amino acid instead of L-amino acid.

In another preferred embodiment of the invention the peptidomimetic variants of linear or cyclic peptide having SEQ ID NO:2 or SEQ ID NO:4 are selected from the group consisting of:

βA-G-R-L-(D)R-(D)W-L-R-V (SEQ ID NO:15)
βA-G-R-L-(D)R-W-D(L)-R-V (SEQ ID NO:16)
βA-G-(D)R-L-(D)R-(D)W-L-R-V (SEQ ID NO:17)
βA-G-R-L-R-(D)W-(D)L-R-V (SEQ ID NO:18)
βA-G-R-L-R-(D)W-L-(D)R-V (SEQ ID NO:19)
βA-G-(D)R-L-R-(D)W-L-R-V (SEQ ID NO:20)
βA-G-R-L-R-ahomoW-L-R-V (SEQ ID NO:21)
βA-G-R-L-R-W-aL-R-V (SEQ ID NO:22)
βA-G-R-L-R-hW-L-R-V (SEQ ID NO:23)
βA-G-R-L-R-W-hL-R-V (SEQ ID NO:24)
βA-G-R-hL-R-W-L-R-V (SEQ ID NO:25)
wherein aL is aza-Leu; ahomoW is aza-homoTrp, hL is hydrazino-Leu; hW is hydrazino-Trp; and (D) indicates that it is a D-amino acid instead of L-amino acid.

A further object of the invention relates to the use of the linear or cyclic peptide of formula (I) as antimicrobial or fungicide agent in agronomic field.

In a preferred embodiment the linear or cyclic peptides of the invention are advantageously used for treating or preventing the infections mediated by Oomycetes. Preferably, said Oomycetes are selected from the taxonomic families of *Peronosporales* and *Pythiaceae*. More preferably, said Oomycetes belong to *Phytophtora spp., Pythium spp.* and *Peronospora spp.*

According to a preferred embodiment, the linear or cyclic peptide of the invention may be used for treating or preventing *Phytophthora infestans* or *Phytophthora capsici* infection.

In another embodiment, the linear or cyclic peptide of the invention may be used for treating or preventing downy mildew infection caused by *Plasmopara viticola* on vine crops.

Yet in another embodiment the linear or cyclic peptide of the invention may be used for treating or preventing *Phythium ultimum* infection.

A further object of the invention is a phytopharmaceutical composition comprising at least one linear or cyclic peptide of formula (I) as active principle, and optionally a solid or liquid solvent and /or diluent, at least an adjuvant and/or co-formulating agent.

In a particular preferred embodiment, the phytopharmaceutical composition may comprise one or more further active ingredients such as a fungicide other than the linear or cyclic peptide of formula (I), selected from the groups consisting of phytoregulators, antibiotics, herbicides, insecticides, fertilizers and/or mixtures thereof.

The aforesaid phytopharmaceutical composition can be in solid form (such as granules, granules dispersible in water, dry powders etc.) or in liquid form (for example solutions, suspensions, emulsions or microemulsions). According to a preferred embodiment of the present invention, the compositions are in spray formulation.

The total concentration of the active peptide in the aforesaid compositions can vary within a broad range; in general, it varies from 1% to 90% by weight relative to the total weight of the composition, preferably from 5% to 50% by weight relative to the total weight of the composition.

The application of these phytopharmaceutical compositions can take place on each part of the plant, for example on leaves, stems, branches and roots, or on the seeds themselves before sowing, or on the soil in which the plant grows.

The present invention further relates to a method for controlling phytopathogenic fungi in agricultural crops, which consists in applying, on any part of the plants to be protected or on the ground, effective and non-phytotoxic doses of a linear or cyclic peptide of formula (I) used as such or formulated in phytopharmaceutical composition. Said phytopathogenic fungi belong to Oomycetes class and are preferably selected from the group consisting of *Phytophthora infestans, Phytophthora capsici, Plasmopara viticola, Peronospora spp, Phytophtora spp* and *Phythium ultimum*. Said agricultural crops comprise fruit bearing crops, preferably vine, tomato, potato, maize, wheat and horticultural crops.

Moreover, the present invention contemplates a nucleotide sequence encoding for one of the peptides of the invention having formula (I). According to a preferred embodiment said nucleotide sequence encodes for the linear or cyclic peptides of the invention having amino acid sequence SEQ ID NO:1 and SEQ ID NO:2 (CP20 or CP32 peptides and their corresponding linear peptides Ac-LP20 and Ac-LP32).

In a more preferred embodiment, the present invention further relates to a nucleotide sequence selected between:

MGNTGGATGYTNGTNTAYMGNMGN (SEQ ID NO:5) encoding for peptide CP20/Ac-LP20
GGNMGNYTNMGNTGGYTNMGNGTN (SEQ ID NO:6) encoding for peptide CP32/Ac-LP32
wherein A = adenine; C = cytosine; G = guanine; T = thymine; N = A, C, G, T; M = A, C; Y = C, T.

Finally, the present invention relates to a transgenic plant expressing at least one nucleotide sequence or genetic construct encoding for a linear or cyclic peptide of formula (I).

Transgenic plants may be produced using different methodologies, such as Agrobacterium-mediated transformation, particle bombardment, various nanoparticles and virus-based RNA delivery systems.

The present invention will now be described for illustrative but non-limiting purposes, according to a preferred embodiment with particular reference to the attached figures, in which:

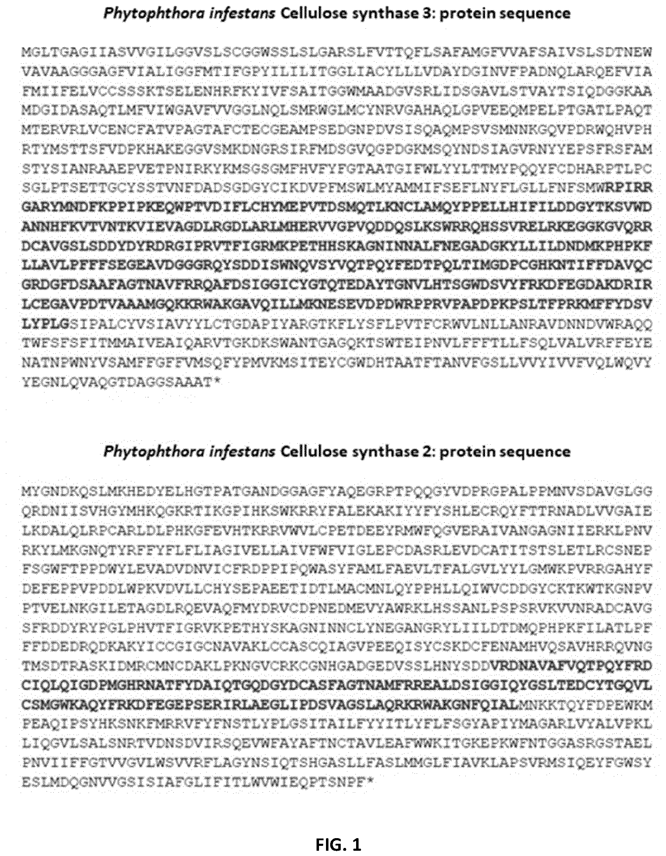

- Figure 1 shows amino acid sequences of *Phytophthora infestans* Cellulose synthase 3 (PiCesA3) and *Phytophthora infestans* Cellulose synthase 2 (PiCesA2) targets with the cloned catalytic domain highlighted in red and used as bait in the yeast two-hybrid assay.

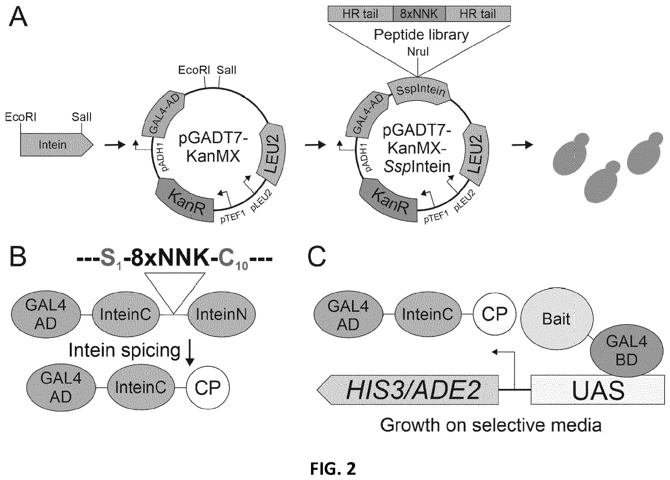

- Figure 2 shows the identification of cyclic peptides. (A) Schematic overview of library construction, the intein gene was inserted into the pGADT7-KanMX vector by means of cohesive ends between insert and vector, obtained by restriction enzymes, and ligation. The library was constructed by homologous recombination between the vector and an oligonucleotide containing the 8xNNK codons flanked by sequences identical to the insertion sites in the vector. Then the oligonucleotide and plasmid were co-transformed in yeast to generate the library. (B) The peptide coding sequence inserted between the two domains InteinC and InteinN is cyclized and, retaining binding to InteinC, can be assayed by yeast two-hybrid strategy as it forms a chimeric protein with the GAL4-AD domain. (C) Yeast two-hybrid assay by which peptides can be selected based on their interaction with a bait target protein. The bait protein is fused to the GAL4-BD binding upstream activator region (UAS) of some reporter genes. If CP is able to physically interact with the bait protein, GAL4-AD and GAL4-BD are brought close together and thus their activity reconstituted, allowing transcription of reporter genes and subsequent growth on selective medium.

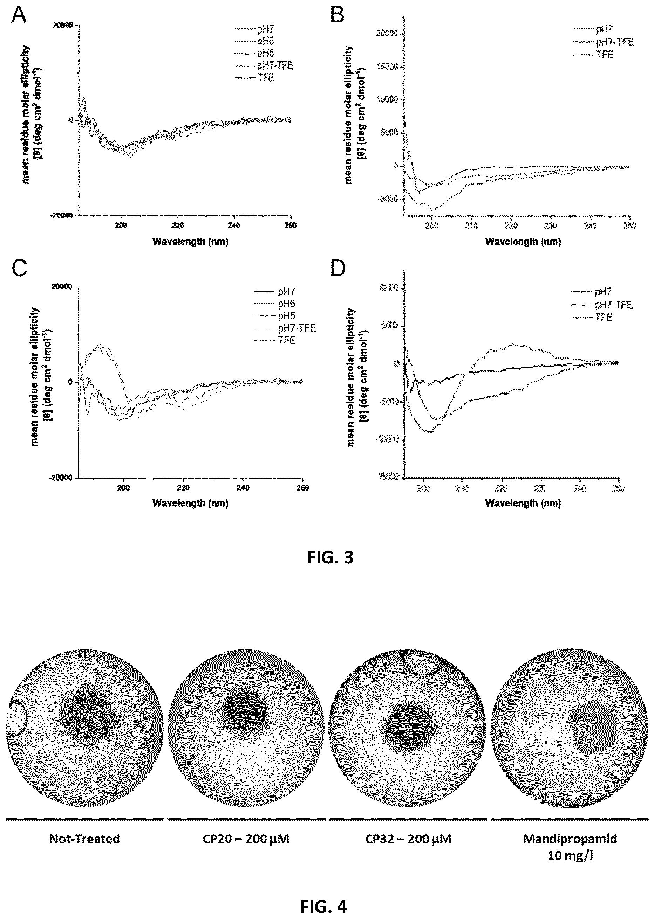

- Figure 3 shows the physical-chemical properties of cyclic and linear peptides determined by circular dichroism in different solutions. A) CP20: 10 mM phosphate buffer, pH7; 10 mM MES buffer, pH6; 10 mM acetate buffer, pH5; 10mM phosphate buffer/TFE (1:1); TFE (trifluoroethanol). B) CP32: Phosphate buffer 10 mM, pH7; Phosphate buffer 10mM/TFE (1:1); TFE (trifluoroethanol). C) Ac-LP20: 10 mM phosphate buffer, pH7; 10 mM MES buffer, pH6; 10 mM acetate buffer, pH5; 10mM phosphate buffer/TFE (1:1); TFE (trifluoroethanol). (D) Ac-LP32: Phosphate buffer 10 mM, pH7; Phosphate buffer 10mM/TFE (1:1); TFE (trifluoroethanol).
- Figure 4 shows images of wells inoculated with mycelium plug (5 mm diameter) of *P. infestans* on culture medium supplemented or not with CP/fungicide.

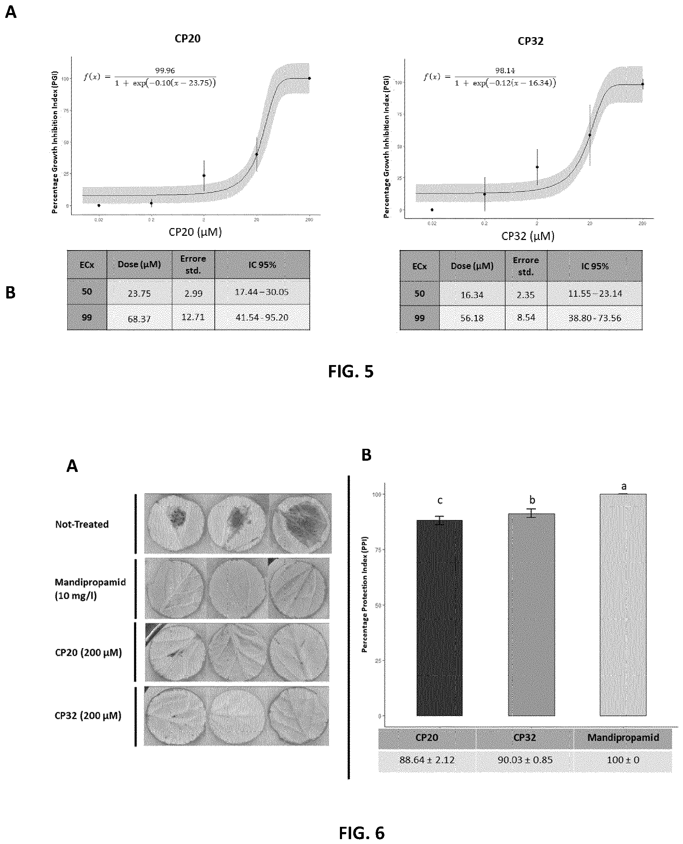

- Figure 5 shows A) Dose-response plots obtained according to the method described by Ritz et al. (2015) [33], employing a three-parameter logistic model. Percent growth inhibition indices (PGIs) were obtained by treating asexual spores (sporangia) of *P. infestans* at different concentrations of peptide (200-20-2-0.2-0.02 $\mu$M). The 95% confidence interval of the model under investigation (IC95) is shown in light gray, and the dots show the mean PGI values and standard deviation (vertical bars). (B) $EC_{50}$ and $EC_{99}$ parameter values estimated from the above logistic model, expressed as $\mu$M dose. Standard error and 95% confidence interval related to the parameter estimation are also shown.

- Figure 6 shows A) Leaf discs of tomato (*Solanum lycopersicum,* cv. Marmande) inoculated with *P. infestans* sporangia ($2 \times 10^4$ sporangia/ml) and subjected to different treatments as shown in the figure. (B) Mean percent protection index compared with the untreated control $\pm$ standard deviation (in the form of error bars in the graph). Letters show significant differences identified by statistical analysis (KW chi-squared = 14.63, df = 2, p-value < 0.001).

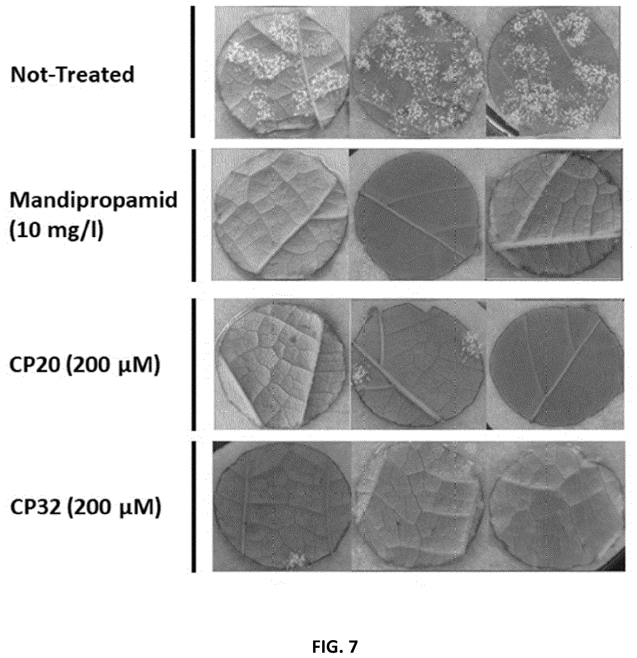

- Figure 7 shows leaf discs of grapevine (Vitis vinifera cv. Pinot noir) inoculated with *P. viticola* sporangia ($1 \times 10^5$ sporangia/ml) and subjected to different treatments.

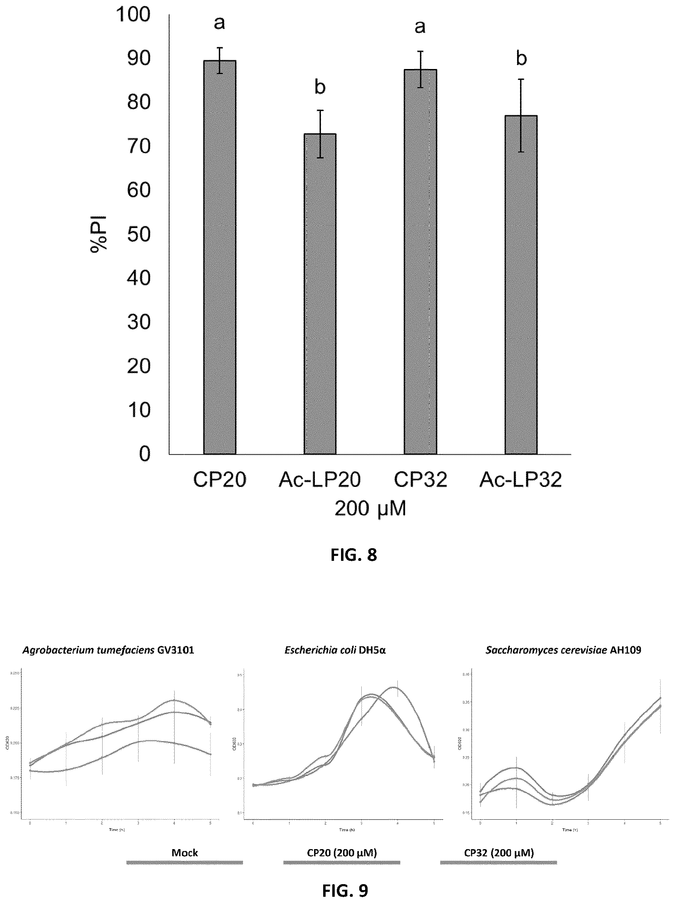

- Figure 8 shows antimicrobial activity of Ac-LP20 and Ac-LP32 peptides. Mean percent protection index versus untreated control $\pm$ standard deviation (in the form of error bars in the graph). Letters show significant differences identified by statistical analysis (Kruskal-Wallis one-way test).

- Figure 9 shows biological activity of CP20 and CP32 peptides against *Agrobacterium tumefaciens, Escherichia coli* and *Saccharomyces cerevisiae.* Peptides were assayed at 200 $\mu$M and growth measured hourly for 5 hours.

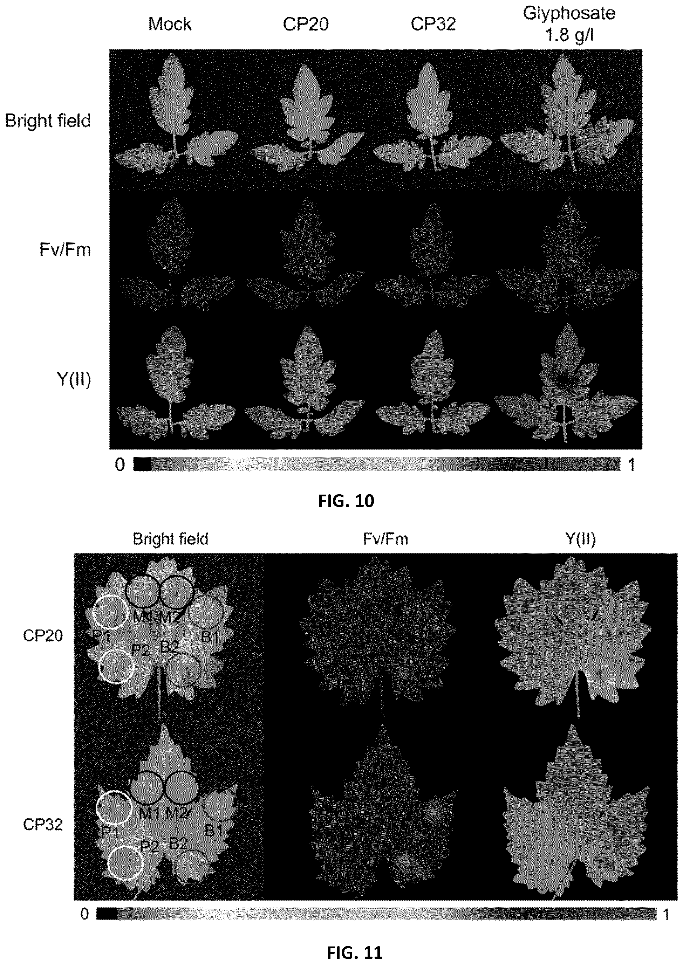

- Figure 10 shows phytotoxicity of CP20 and CP32 peptides. Tomato leaves treated with Mock (water), CP20 (200 $\mu$M), CP32 (200 $\mu$M) and glyphosate 1.8 g/L and incubated for 5 days. After 5 days, the values of Fv/Fm and Y(II) were recorded. Below the image is the colorimetric scale related to photosynthetic efficiency.

- Figure 11 shows phytotoxicity of CP20 and CP32 peptides. The circles shown in the image correspond to where the different treatments were applied on the grapevine leaf surface and the letters correspond to: M1, M2 = water; P1 = CP20 or CP32 at 200 $\mu$M; P2 = CP20 or CP32 at 400 $\mu$M; B1 = BASTA at 200 mg/ml; B2 = BASTA at 400 mg/ml. After treatment, the leaves were incubated for 7 days. After 7 days, the values of Fv/Fm and Y(II) were recorded. Below the image is the colorimetric scale related to photosynthetic efficiency.

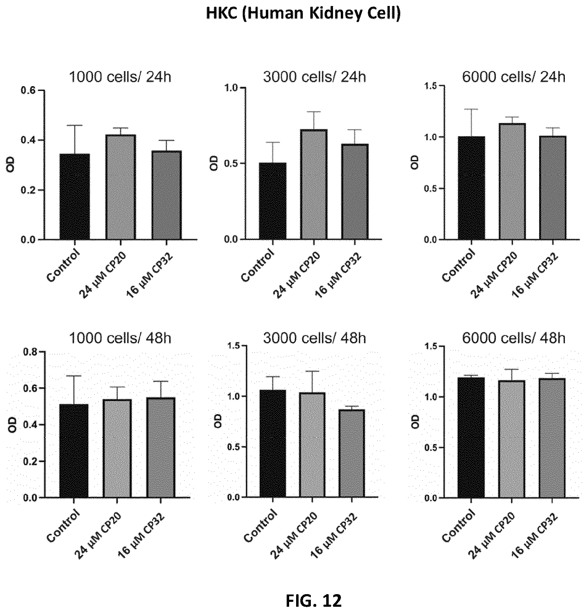

- Figure 12 shows results of MTT assay of cytotoxicity of CP20 and CP32 peptides on human HKC cells. The peptides were used at their respective $EC_{50}$ concentrations (16 $\mu$M CP32 and 24 $\mu$M CP20), and absorption at 570 nm (cell viability signal) was measured after 24 and 48 hours. OD, Optical density.

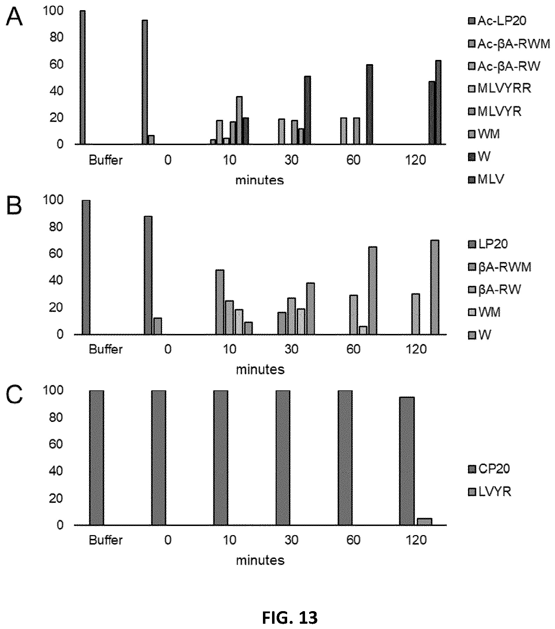

- Figure 13 shows percentage of Ac-LP20 (A), LP20 (B), CP20 (C) and their respective degradation forms following their incubation with a protease mixture for different time periods. Ac-LP20/CP20 sequence: βA-R-W-M-L-V-Y-R-R (SEQ ID NO:1).

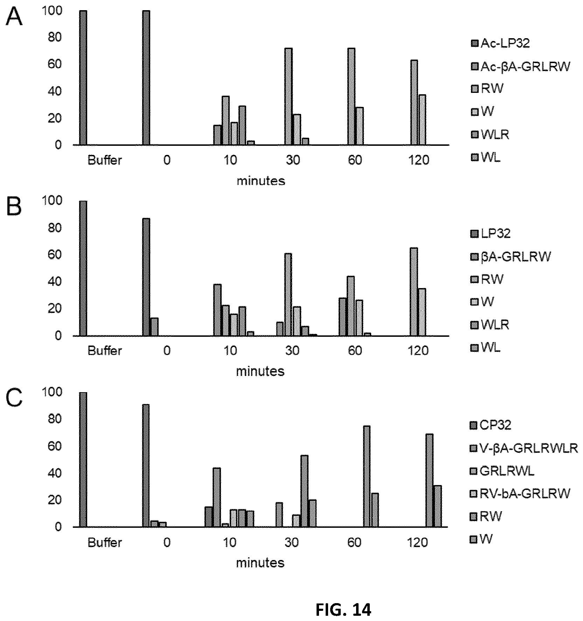

- Figure 14 shows percentage of Ac-LP32 (A), LP32 (B), CP32 (C) and their respective degradation forms following their incubation with a protease mixture for different time periods. Ac-LP32/CP32 sequence: βA-G-R-L-R-W-L-R-V (SEQ ID NO:2).

The following non-limiting examples are now provided for a better illustration of the biological activity of the cyclic and linear antimicrobial peptides of the invention on plates and leaves in vitro with promising results.

**EXAMPLES**

Example 1: *Identification of the cyclic peptides CP20 and CP32*

Peptides CP20 and CP32 were selected by means of a yeast two-hybrid assay with the aim of identifying cyclic peptides, within a peptide library of 8 amino acids [13], capable of physically interacting with the catalytic domain of Cellulose synthase A3 (PiCesA3) and the catalytic domain of Cellulose synthase A2 (PiCesA2) of *P. infestans* (Figure 1).

Indeed, it has been shown in *P. infestans* and *P. viticola* that enzymes of the cellulose synthase family play a key role in the primary infection of the host plant, as they contribute to the germination of the zoospores and the subsequent formation of the germination tube necessary to overcome the host's external protective barriers [14, 15].

In addition, the cellulose synthases of oomycetes have proven to be excellent candidates for the development of antimicrobials, as they differ highly in amino acid sequence from all other cellulose synthases known outside the taxonomic class of oomycetes [16]. The peptide library was constructed following the protocol reported in Rosa et al. [13], with the aim of testing it using yeast two-hybrid technology, based on the possibility of being able to split the yeast transcription factor GAL4 into two independent portions: the transcription activation domain (GAL4-AD) and the DNA-binding domain (GAL4-BD). The combinatorial library was obtained using a degenerate oligonucleotide containing the eight-fold repeated NNK codon (8$\times$NNK) and exploits the ability of engineered inteins to produce lariat peptides (lariat refers to peptides that are cyclized by the formation of an ester between a side chain and a C-terminal) in vivo [18]. Specifically, the library construction strategy involved:

(i) The cloning of the synthetic gene encoding Intein into the pGADT7-KanMX vector (pGADT7-KanMX-SspIntein) fused to the GAL4-AD domain (Figure 2A) [12];

(ii) The homologous recombination of the combinatorial library between the C-terminal domain (InteinC) and the N-terminal domain (InteinN) of Intein (InteinC-8xNNK-InteinN), which, as shown in Figure 2, are reversed from their natural order (InteinN-InteinC) [19]. The resulting library obtained by homologous recombination is capable of producing a chimeric protein with the InteinC and InteinN domains flanking the combinatorial library and catalysing the cyclization of the peptide encoded by the 8xNNK codons (Figure 2B). It should be noted that the lariat peptide manages to maintain a bond with InteinC, which is itself fused to the GAL4-AD domain, which is essential to perform the yeast two-hybrid assay;

(iii) The library was then used to transform yeast strain AH109 (Clontech, (MATa, trp1-901, leu2-3, 112, ura3-52, his3-200, gal4Δ, gal80Δ, LYS2::GAL1UAS-GAL1TATAHIS3, GAL2UAS-GAL2TATA-ADE2, URA3::MEL1 UASMEL1TATA-lacZ, MEL1) (Figure 2A). The pGBKT7 vector was then used to express in yeast the catalytic domains of the enzymes PiCesA2 and PiCesA3 fused with the GAL4-BD domain, to be used as bait in the yeast two-hybrid assay. Such vectors were individually transformed into the yeast strain Y187 (MATα, ura3-52, his3-200, ade2-101, trp1-901, leu2-3, 112, gal4Δ, met-, gal80Δ, URA3::GAL1UAS-GAL1TATA-lacZ) and co-cultured with the library-transformed AH109 cells previously stored at -80 °C. This made it possible to generate diploid yeast cells with both the plasmid encoding the GAL4-BD-fusion and the library plasmid expressing one of the CPs contained therein fused to GAL4-AD, bringing together all the necessary elements for the yeast two-hybrid assay. Cyclic peptides capable of interacting with the two catalytic domains were selected based on the ability of the positive yeast clones to grow on a selective medium (Figure 2C). The plasmid DNA containing the 24-nucleotide fragments of the combinatorial library within the positive clones was then amplified by PCR and sequenced, allowing the primary structure of the CP20 and CP32 peptides to be deduced.

Example 2: *Synthesis of the cyclic peptides CP20 and CP32, their linear versions Ac-LP20, Ac-LP32, LP20, LP32 and their purification*

Cyclic peptides and their linear versions were prepared by solid-phase peptide synthesis with an automated, microwave-assisted Fmoc/tBu strategy [20].

In the case of the cyclic peptides CP20 and CP32, 2-chlorotrityl chloride resin was used as a solid support, with a loading of 0.3-0.6 mmol/g and on a scale of 0.1 mmol. Coupling was performed using 5 equivalents of the corresponding amino acid, previously dissolved in DMF to obtain a 0.2 M solution. DIC (0.5 M in DMF) and Oxyma Pure (1M in DMF), to which DIPEA (0.1M) was added, were used as coupling reagents. A 20% piperidine solution in DMF was used for the Fmoc group deprotection step. The coupling reaction was conducted at 25°C for 120 s, followed by 480 s at 50°C and 35W. For arginine, a double coupling was carried out. For the detachment of the Fmoc group, a standard deprotection was performed: at 75°C, 155 W for 15 s, followed by 60 s at 90°C, 50 W.

Detachment of the peptide from the solid support was performed by reacting the resin with a DCM/TFE/AcOH solution (8:1:1) for 2 h at room temperature. The solution was then filtered, transferred into a flask and evaporated under reduced pressure. The product was precipitated in water and then lyophilized for subsequent cyclization. 1 equivalent of the protected linear peptide was dissolved in DMF (0.2 mM) and DIPEA was added to pH 8. Under these conditions, BOP (6 eq) and HOBt (6 eq) were added, and the reaction was left to stir at room temperature.

After 6 hours, BOP was added again and the pH checked and if necessary adjusted with DIPEA. The reaction was left to stir for 16 hours. The next day, the solvent was evaporated under reduced pressure and the crude was dissolved in ethyl acetate and subjected to a first purification step by extraction to remove coupling reagents and residual DMF. The organic phase containing the protected peptide was evaporated and the dry peptide subjected to cleavage.

Detachment of the protecting groups was performed by reacting the peptide with the TFA/phenol/water/TAN/-DODT cocktail, 82.5:5:5:5:2.5 v/v for 3h. The peptide was precipitated from cold ethyl ether and purified by RP-HPLC with a 15-70% gradient of solvent B (solvent A: water/TFA 100/0.1; solvent B: acetonitrile/TFA 100/0.1) in 20 min at a flow rate of 20 ml/min. The peptide was lyophilized and stored at -15°C.

In the case of the linear peptides Ac-LP20, Ac-LP32, LP20 and LP32, Rink Amide resin was used as a solid support with a loading of 0.7 mmol/g and on a scale of 0.1 mmol. Coupling was performed using 5 equivalents of the corresponding amino acid, previously dissolved in DMF to obtain a 0.2 M solution. DIC (0.5 M in DMF) and Oxyma Pure (1M in DMF) were used as coupling reagents. For the Fmoc group deprotection step, a 20% piperidine solution in DMF was used. A standard protocol was followed for the coupling reaction: 15s at 75°C, 170W followed by 110s at 90°C, 40W. For arginine, a double coupling was performed. For Fmoc coupling, a standard deprotection was performed: 15s at 75°C, 155W, followed by 60s at 90°C, 50W. Following the last deprotection, the peptide was acetylated by adding a mixture of Ac2O/DIPEA/DCM (1:1:8) to the resin, reacted for 15 minutes and then removed. Detachment from the resin and the protective groups of the amino acid residue side chains was performed by reacting the resin with a cocktail of TAN, DODT and TFA (7:3:90) for 3h. The peptide was precipitated from cold diethyl ether and purified by RP-HPLC with a 15-70% gradient of solvent B (solvent A: water/TFA 100/0.1; solvent B: acetonitrile/TFA 100/0.1) in 20 min at a flow rate of 20 ml/min. The peptide was lyophilized and stored at -15°C.

Example 3: *Chemical-physical properties of the cyclic peptides CP20 and CP32 and their linear versions Ac-LP20 and Ac-LP32*

CP20 is a head-tailed cyclic peptide consisting of a β-amino acid and 8 L-amino acids:

β Ala - Arg - Trp - Met - Leu - Val - Tyr - Arg - Arg (SEQ ID NO: 1)

CP20 has a molecular weight of 1232.5 Da, an isoelectric point at pH 12.4, a net charge of +3 at pH 7, and good solubility in water with the following amino acid composition: 55.56% hydrophobic, 33.33% basic, 11.11% neutral and 0% acid. Conformational studies based on circular dichroism were performed under different conditions, such as:

- 10 mM phosphate buffer, pH 7
- MES buffer 10 mM, pH 6
- Buffer acetate 10 mM, pH 5
- Phosphate buffer 10 mM/TFE (1:1)
- TFE (trifluoroethanol)

In general, the structure develops between random coil and β-strand both under aqueous conditions and with the addition of TFE (Figure 3A and Table 1).

**Table 1.** Percentages related to the different conformations of CP20 found in the different solutions. As can be seen, the structure develops between random coil and β-strand in both aqueous conditions and with the addition of TFE.

| Conditions | Helixes (%) | Sheets (%) | Turns (%) | Disordered (%) |
|---|---|---|---|---|
| pH 7 | 6 | 40 | 22 | 32 |
| pH 6 | 8 | 37 | 21 | 34 |
| pH 5 | 7 | 39 | 23 | 31 |
| pH 7-TFE | 8 | 36 | 22 | 34 |
| TFE | 10 | 35 | 22 | 33 |

CP32 is a head-tailed cyclic peptide consisting of a β-amino acid and 8 L-amino acids:

β Ala - Gly - Arg - Leu - Arg - Trp - Leu - Arg - Val (SEQ ID NO:2)

CP32 has a molecular weight of 1108.4 Da, an isoelectric point at pH 14, a net charge of +3 at pH 7, and good solubility in water with the following amino acid composition: 55.56% hydrophobic, 33.33% basic, 11.11% neutral and 0% acid. Conformational studies based on circular dichroism were performed under different conditions such as:

- 10 mM phosphate buffer, pH7
- Phosphate buffer 10mM/TFE, pH7 (1:1)
- TFE (trifluoroethanol)

In general, the structure develops between random coil and β-strand both in aqueous solution and with the addition of TFE (Figure 3B and Table 2).

**Table 2.** Percentages related to the different conformations of CP32 found in the different solutions. As can be seen, the structure develops between random coil and β-strand both in aqueous solution and with the addition of TFE.

| Conditions | Helixes (%) | Sheets (%) | Turns (%) | Disordered (%) |
|---|---|---|---|---|
| pH 7 | 5 | 34 | 18 | 43 |
| pH 7-TFE | 5 | 33 | 18 | 44 |
| TFE | 6 | 29 | 18 | 47 |

Ac-LP20 is a linear peptide with a C-terminal amide group consisting of a β-amino acid and 8 L-amino acids:

Ac-βAla - Arg - Trp - Met - Leu - Val - Tyr - Arg - Arg $CONH_2$ (SEQ ID NO:3)

Ac-LP20 has a molecular weight of 1291.6 Da, an isoelectric point at pH 12.4, a net charge of +3 at pH 7, and good solubility in water with the same amino acid composition as CP20.

Conformational studies based on circular dichroism were performed under different conditions, such as:

- 10 mM phosphate buffer, pH7
- MES buffer 10 mM, pH6
- Buffer acetate 10 mM, pH5
- Phosphate buffer 10 mM/TFE, pH7 (1:1)
- TFE (trifluoroethanol)

In general, the structure develops between random coil and β-strand under aqueous conditions, while the tendency to form an α-helix increases with the addition of TFE (Figure 3C and Table 3).

**Table 3.** Relative percentages of the different conformations of Ac-LP20 found in the different solutions. As can be seen, the structure develops between random coil and β-strand under aqueous conditions, while the tendency to form an α-helix increases with the addition of TFE.

| Conditions | Helixes (%) | Sheets (%) | Turns (%) | Disordered (%) |
|---|---|---|---|---|
| pH 7 | 5 | 36 | 22 | 37 |
| pH 6 | 6 | 40 | 21 | 33 |
| pH 5 | 7 | 35 | 22 | 36 |
| pH 7-TFE | 18 | 29 | 24 | 29 |
| TFE | 19 | 27 | 23 | 31 |

Ac-LP32 is a linear peptide with a C terminal amide group consisting of a β-amino acid and 8 L-amino acids: Ac-βAla - Gly - Arg - Leu - Arg - Trp - Leu - Arg - Val - CONH$_2$ (SEQ ID NO:4)

Ac-LP32 has a molecular weight of 1167.4 Da, an isoelectric point at pH 14, a net charge of +3 at pH 7, and good solubility in water with the same amino acid composition as CP32. Conformational studies based on circular dichroism were performed under different conditions, such as:

- 10 mM phosphate buffer, pH7
- 10mM phosphate buffer/TFE (1:1)
- TFE (trifluoroethanol)

In general, the structure develops between random coil and β-strand both in aqueous solution and with the addition of TFE (Figure 3D and Table 4).

**Table 4.** Percentages related to the different conformations of Ac-LP32 found in the different solutions. As can be seen, the structure develops between random coil and β-strand both in aqueous solution and with the addition of TFE.

| Conditions | Helixes (%) | Sheets (%) | Turns (%) | Disordered (%) |
|---|---|---|---|---|
| pH 7 | 5 | 33 | 18 | 44 |
| pH 7-TFE | 5 | 30 | 18 | 47 |
| TFE | 5 | 30 | 14 | 51 |

Example 4: *Antimicrobial activity of CP20 and CP32 peptides*

The two cyclic peptides CP20 and CP32 were tested to determine their antimicrobial efficacy against the pathogen *P. infestans*. Different testing methods were used for this purpose, both *in vitro* on culture medium under axenic conditions and on tomato leaf disc following inoculation. The reference concentration used to evaluate the activity of the two cyclic peptides was 200 μM. Subsequently, the effective concentrations EC$_{50}$ (peptide concentration such as to produce 50% of the maximum antimicrobial effect found) and EC$_{99}$ (peptide concentration such as to produce 99% of the maximum antimicrobial effect found) were determined by dose/response analysis.

To make the results obtained by the different experimental methodologies comparable, the results obtained were expressed as percentage efficacy indexes expressing the reduction in pathogen growth (axenic culture assays) or symptoms caused by the pathogen (leaf disc assays), compared to the negative control (not treated with the peptide/-fungicide) (see below Table 5).

Table 5. Formulas for calculating the different percentage effectiveness indices used in the different antimicrobial assays.

| Effectiveness index | Formula | Input variables |
|---|---|---|
| (1) Percentage Protection Index (PPI) (Petri dish - solid medium) | $$PPI = 100 - \frac{(D_1 \times 100)}{D_2}$$ | $D_1$: measured diameter (in cm or mm) for colonies treated with fungicide/aptamer; $D_2$: mean diameter measured for untreated colonies. |
| (2) Percentage Growth Inhibition Index (PGI) (Petri dish - liquid medium) | $$PGI = 100 \times \frac{[(At_{fNT} - At_{0NT}) - (At_{fT} - At_{0T})]}{[(At_{fNT} - At_{0NT})]}$$ | $At_f$: absorbance measured at end time; $At_0$: absorbance measured at end time zero; NT: not treated with fungicide/aptamer; T: treated with fungicide/aptamer. |
| (3) Percentage Protection Index (PPI) (Leaf disc) | $$PPI = 100 - \frac{(DSI_1 \times 100)}{DSI_2}$$ | $DSI_1$: Disease severity index of the discs treated with fungicide/aptamer; $DSI_2$: Average disease severity |

In order to verify the existence of significant differences between the efficacy indices for the cyclic peptides under investigation and the positive control (fungicide treatment, mandipropamid; Syngenta, Italy), a Kruskal-Wallis test was performed.

In the presence of significant differences (p-value < 0.05), a multiple comparison was performed by means of Fisher's LSD test with Benjamini-Hochberg correction.

*Agarised medium assays inoculated with mycelium*

The peptides CP20 and CP32 were added separately to pre-warmed (50°C) agar medium (PAM, Pea Agar Medium) until the final concentration of interest was reached. Subsequently, 1 ml of medium was dispensed into each well of a 24-well culture plate. Four wells (biological repeats) were used for each treatment, including a negative control with sterile distilled water and a positive control containing the commercial fungicide (mandipropamid 10 mg/l; Syngenta). Each well was inoculated using a mycelium plug (5 mm in diameter) taken from the margin of an actively growing *P. infestans* colony. After the incubation period (3-4 days), 3 diameters of growth were measured for each colony, followed by calculation of the Percentage Protection Index (PPI; Table 5) compared to the untreated control. Both peptides assayed at 200 $\mu$M caused a clear reduction in mycelial growth (Figure 4), with PPI values above 85% (see below Table 6).

Table 6. Percentage efficacy indices $\pm$ standard deviation, related to solid medium (PPI - Percent Protection Index) and liquid medium (PGI - Percent Growth Inhibition Index) assays for the different treatments. The letters show the significant differences identified by statistical analysis.

| Treatment | PPI (%) $\pm$ dev. std (Solid medium)[1] | PGI (%) $\pm$ dev. std (Liquid medium)[2] |
|---|---|---|
| CP20 - 200 $\mu$M | 88.99 $\pm$ 7.56 (b) | 86.19 $\pm$ 8.66 (b) |
| CP32 - 200 $\mu$M | 87.93 $\pm$ 9.47 (b) | 90.61 $\pm$ 10.87 (b) |
| Mandipropamid - 10 mg/l | 100.00 $\pm$ 0 (a) | 100.00 $\pm$ 0 (a) |
| [1] Kruskal-Wallis chi-squared = 24.08, df = 2, p-value < 0.001 [2] Kruskal-Wallis chi-squared = 25.68, df = 2, p-value < 0.001 | | |

*Assays on liquid medium inoculated with asexual spores (sporangia)*

The peptides CP20 and CP32 were added separately to liquid culture medium (PB, Pea Broth) until the final concentration of interest was reached. Subsequently, 50 $\mu$l of medium was dispensed into each well of a 96-well, flat-bottomed culture plate. Eight wells (biological repeats) were used for each treatment, including a negative control with sterile distilled water medium and a positive control containing the commercial fungicide (mandipropamid 10 mg/l; Syngenta). Each well was inoculated using a suspension of sporangia (at a concentration of $2 \times 10^4$ sporangia/ml) taken from axenic cultures of *P. infestans.* Mycelial growth was assessed by spectrophotometric absorbance measurement ($OD_{620}$), performed every 24 h from inoculation for 72 h. Next, the Percentage Growth Inhibition Index (PGI; Table 5) was calculated compared to the untreated control. Again, the efficacy of the two peptides was very high, with PGI values above 85% for both molecules when tested at 200 $\mu$M (see Table 6).

In order to determine the dose/response relationship of the two cyclic peptides, sporangia were assayed at five different peptide concentrations (0.02, 0.2, 2, 20, 200 $\mu$M) and growth measured using the methods listed above. The effective concentrations ($EC_{50}$ and $EC_{99}$) were determined from the PGI values, using a three-parameter logistic model (Figure 5) according to the method proposed by Ritz et al. [33]. In both cases, the $EC_{99}$ value (Figure 5) was less than 70 $\mu$M ($EC_{99}$: 68.37 $\mu$M for CP20 and 56.18 $\mu$M for CP32).

*Co-inoculation assays on leaf tissue*

Leaf discs with a diameter of 1.5 cm obtained from tomato (*Solanum lycopersicum,* cv. Marmande) leaves were inoculated by placing 10 $\mu$l of a suspension of *P. infestans* sporangia (at a concentration of $2 \times 10^4$ sporangia/ml) containing, or not, the peptide at the final concentration of interest. Nine leaf discs were inoculated for each treatment. The discs, placed in Petri dishes (9 cm diameter) with moistened filter paper were incubated in a growth chamber at a temperature of 22 $\pm$ 1 °C with a photoperiod of 12 hours light and 12 hours dark. After the incubation period (5-7 days), the severity of the disease was assessed by assigning a class to each leaf disc relative to the percentage of leaf disc area with symptoms. This was followed by calculation of the Percentage Protection Index for the leaf discs (PPI; Table 5) compared to the untreated control. Again, the two peptides showed a high inhibition capacity against the pathogen, showing a reduction in symptoms equal to (CP32: 90.03%) or very close to (CP20: 88.64%) 90% (Figure 6).

*Evaluation of the efficacy of CP20 and CP32 against other phytopathogenic oomycetes*

In view of the high protein sequence similarity of the target enzymes (Cellulose synthase A - CesA) of the genera Phytophthora [21, 22, 23] and other Peronosporales [24], the peptides CP20 and CP32 were tested against other oomycete species, including: *Phytophthora capsici* Leonian, *Pythium ultimum* var. *ultimum* Trow and *Plasmopara viticola* (Berk. et Curt.) Berl. and De Toni. In order to evaluate the efficacy of CP20 and CP32 against the above-mentioned species, the previously described assays were used as follows:

(i) *P. capsici*: activity against *P. capsici* was assessed by means of the liquid medium assay inoculated with asexual spores (sporangia) previously described, the only modification made being the culture medium used (PB, Potato Dextrose Broth);

ii) *P. ultimum:* activity against *P. ultimum* was assessed by means of the agarised medium inoculated with mycelium previously described, the only modification being the culture medium used (PDA, Potato Dextrose Agar);

iii) *P. viticola*: activity against *P. viticola* was assessed by means of the co-inoculation assay on leaf tissue previously described, with some modifications.

In fact, grapevine leaf discs (*Vitis vinifera* cv. *Pinot noir*) were used, inoculated by placing five drops (10 $\mu$l each) of a suspension of *P. viticola* sporangia (at a concentration of $1 \times 10^5$ sporangia/ml; see also Colombo et al. [23]) on them. 24 hours after inoculation, the drops were removed with filter paper.

Both peptides showed high inhibition values against the three oomycete species under investigation (Table 7).

**Table** 7. Percentage efficacy indices related to the evaluation of the spectrum of action of the two peptides CP20 and CP32 against other phytopathogenic oomycete species (*P. capsici, P. ultimum* and *P. viticola*). Results are reported as mean percent protection index compared with the untreated control $\pm$ standard deviation. The letters show the significant differences identified by statistical analysis.

| Treatment | Target enzyme | Target species | Assayed species | | |
|---|---|---|---|---|---|
| | | | *P. capsici*[1] (PIC - %) | *P. ultimum*[2] (IPP - %) | *P. viticola*[3] (IPP - %) |
| CP20 | PiCesA3 | *P. infestans* | 100 $\pm$ 0 (a) | 75.98 $\pm$ 4.54 (c) | 89.93 $\pm$ 11.69 (b) |
| CP32 | PiCesA2 | *P. infestans* | 89.25 $\pm$ 7.35 (b) | 85.42 $\pm$ 7.28 (b) | 94.92 $\pm$ 6.45 (b) |

(continued)

| Treatment | Target enzyme | Target species | Assayed species | | |
|---|---|---|---|---|---|
| | | | P. capsici[1] (PIC - %) | P. ultimum[2] (IPP - %) | P. viticola[3] (IPP - %) |
| Fungicide* | n.d. | n.d. | 100 ± 0 (a) | 100 ± 0 (a) | 100 ± 0 (a) |
| *Mandipropamid (P. capsici e P. viticola); Metalaxyl-M (P. ultimum) <br> [1]: KW chi-squared = 26.27, df = 2, p-value < 0.001 <br> [2]: KW chi-squared = 10.74, df = 2, p-value = 0.005 <br> [3]: KW chi-squared = 7.61, df = 2, p-value = 0.022 | | | | | |

In general, the efficacy values ranged from 75% (CP20 vs P. ultimum) to 100% (CP20 vs P. capsici), and from 85% (CP32 vs P. ultimum) to 95% (CP32 vs P. viticola; see also Figure 7).

*Antimicrobial activity of Ac-LP20, Ac-LP32 and their Ala-scans*

The antimicrobial activity of the linear analogues of CP20 and CP32, Ac-LP20 and Ac-LP32 (sequence, characteristics and properties presented in Example 3), was assayed at 200 $\mu$M against P. infestans mycelium in the manner previously described. The results indicate that although there was a statistically significant reduction in PPIs compared to their cyclic counterparts (CP20 89.53 % ± 2.96 %, CP32 87.44 % ± 4.19 %), the linear peptides were within efficacy values of 72.78 % ± 5.41 (LP20) and 76.97 % ± 8.2 (LP32; Figure 8). In each case, both show a PPI > 70 %, and can in any case be considered a good starting point for further characterization and possible improvement of the amino acid sequence with different strategies, such as peptidomimetic and formulation.

Once the antimicrobial activity of Ac-LP20 and Ac-LP32 had been verified, they were modified by substituting alanine at different positions in the sequence (Ala-scan) of both peptides. The Ala-scans of Ac-LP20 and Ac-LP32 were tested on mycelium in the manner previously described to verify the relevance of the different amino acids to the antimicrobial activity of the two peptides. In addition, a version of CP20 was also tested where methionine was replaced by an alanine (CP20-Met-Ala) to limit its oxidation.

From the results shown in Table 8, it can be seen that some substitutions cause a major decrease in the effect observed for Ac-LP20 and Ac-LP32 (Ac-LP20-Ala7, -Ala8 and -Ala9; Ac-LP32-Ala8 and -Ala9), or even some Ala-scans show higher PPI values than their starting versions (Ac-LP20-Ala3; Ac-LP32-Ala2). CP20-Met-Ala maintains values above 80% PPI, indicating the low importance of methionine at that peptide position.

Table 8. Obtained values of PPI (%) by treating P. infestans mycelium in vitro with Ala-scan versions of Ac-LP20 and Ac-LP32, as well as CP20-Met-Ala. Highlighted by red font are substitutions with alanine. Letters show significant differences identified by statistical analysis (Kruskal-Wallis one-way test).

| Peptide | PPI (%) mycelium | CLD | Peptide | PPI (%) micelio | CLD |
|---|---|---|---|---|---|
| Ac-LP20 (Ac-$\beta$A-RWMLVYRR-CONH$_2$) SEQ ID NO:3 | 78.80 ± 4.07 | b | Ac-LP32 (Ac-$\beta$A-GRLRWLRV-CONH$_2$) SEQ ID NO:4 | 70.23 ± 3.84 | b |
| CP20-Met-Ala ($\beta$A-RWAL-VYRR) SEQ ID NO:26 | 82.5 ± 5.83 | b | Ac-LP32-Ala2 (Ac-$\beta$A-ARLRWLRV-CONH$_2$) SEQ ID NO:34 | 74.81 ± 3.63 | a |
| Ac-LP20-Ala2 (Ac-$\beta$A-AWMLVYRR-CONH$_2$) SEQ ID NO:27 | 38.75 ± 7.72 | d | Ac-LP32-Ala3 (Ac-$\beta$A-GALRWLRV-CONH$_2$) SEQ ID NO:35 | 58.78 ± 3.94 | c |
| Ac-LP20-Ala3 (Ac-$\beta$A-RAMLVYRR-CONH$_2$) SEQ ID NO:28 | 100 ± 0 | a | Ac-LP32-Ala4 (Ac-$\beta$A-GRARWLRV-CONH$_2$) SEQ ID NO:36 | 56.49 ± 10.10 | c |
| Ac-LP20-Ala5 (Ac-$\beta$A-RWMAVYRR-CONH$_2$) SEQ ID NO:29 | 70.73 ± 0 | c | Ac-LP32-Ala5 (Ac-$\beta$A-GRLAWLRV-CONH$_2$) SEQ ID NO:37 | 40.46 ± 1.76 | d |

(continued)

| Peptide | PPI (%) mycelium | CLD | Peptide | PPI (%) micelio | CLD |
|---|---|---|---|---|---|
| Ac-LP20-Ala6 (Ac-βA-RWMLAYRR-CONH₂) SEQ ID NO:30 | 62.2 ± 7.54 | c | Ac-LP32-Ala6 (Ac-βA-GRLRALRV-CONH₂) SEQ ID NO:38 | 54.20 ± 4.31 | c |
| Ac-LP20-Ala7 (Ac-βA-RWMLVARR-CONH₂) SEQ ID NO:31 | 17.48 ± 12.34 | e | Ac-LP32-Ala7 (Ac-βA-GRLRWARV-CONH₂) SEQ ID NO:39 | 59.29 ± 7.05 | c |
| Ac-LP20-Ala8 (Ac-βA-RWMLVYAR-CONH₂) SEQ ID NO:32 | 10.57 ± 9.49 | f | Ac-LP32-Ala8 (Ac-βA-GRLRWLAV-CONH₂) SEQ ID NO:40 | 17.56 ± 7.48 | e |
| Ac-LP20-Ala9 (Ac-βA-RWMLVYRA-CONH₂) SEQ ID NO:33 | 10.57 ± 9.49 | f | Ac-LP32-Ala9 (Ac-βA-GRLRWLRA-CONH₂) SEQ ID NO:41 | 19.85 ± 2.92 | e |

This information will be useful in the future to be able to introduce further modifications to improve certain properties of Ac-LP20 and Ac-LP32 without negatively affecting their antimicrobial activity.

Example 5: *Specificity of CP20 and CP32*

The specificity of antimicrobial molecules is a key aspect in their development, as good degrees of specificity result in the absence of toxicity to other organisms in the environment such as animals, plants, fungi, bacteria, yeasts, livestock and humans. Therefore, initial assays were carried out to assess the toxicity profiles of CP20 and CP32, similar to those reported in Colombo et al. [15]. For this purpose, the two cyclic peptides were added at a concentration of 200 $\mu$M to liquid cultures of the following microorganism species:

(i) *Agrobacterium tumefaciens* GV3101, (ii) *Escherichia coli* DH5$\alpha$ and (iii) *Saccharomyces cerevisiae.* The culture media used were respectively Luria-Bertani (LB) for the bacterial cultures, while Yeast Extract Peptone Dextrose (YEPD) medium was used for *S. cerevisiae.* Growth was measured hourly (up to 5 h) using a spectrophotometer ($OD_{620}$). The addition of the cyclic peptides to the culture medium caused no alteration in bacterial growth (Figure 9), indicating that the use of the two molecules has no undesirable bacteriostatic or bactericidal effects. Treatment with CP20 and CP32 also showed no growth inhibition against the eukaryotic organism *S. cerevisiae* (Figure 9).

To assess possible phytotoxicity by the two cyclic peptides, CP20 and CP32 were applied to leaf tissue of *Solanum lycopersicum* (tomato) cv. Marmande (Figure 10) and *Vitis vinifera* (grapevine) cv. *Pinot noir* (Figure 11) and after 5 and 7 days, respectively, the leaves were analyzed. Photosynthetic efficiency was assessed using the IMAGING-PAM instrument (Walz, Germany) by measuring the parameters of maximum photo system II quantum yield (Fv/Fm) and effective quantum yield [Y(II)], as they represent good parameters of leaf fitness and have already been used to assess the toxicity of antimicrobial peptides in our group in the past [25]. The different timing was calibrated on the basis of the number of days to allow the damage caused by the positive control (herbicide treatment) to be clearly appreciated only by photosynthetic efficiency measurements, while little or no visible lesions were present in the bright field images, thus demonstrating the sensitivity of the method used.

The cytotoxicity of the cyclic peptides CP20 and CP32 on human kidney proximal tubular cells (HKC-8) was tested in vitro by means of an MTT assay. This assay makes it possible to measure the activity of the mitochondrial enzyme succinate dehydrogenase, which is only active in live cells, and which is capable of reducing MTT (3-[4,5-dimethylthiazol-2-yl]-2,5-diphenyl tetrazolium bromide, yellow in color) to formazan, the formation of which can be followed by measuring absorption at 570 nm. Thus, formazan and absorbance levels at 570 nm are directly proportional to the amount of viable cells present in the sample. Cultures of immortalized human cells (HKC8) at different densities (1000, 3000 and 6000 cells per 100 $\mu$l) were grown in DMEM-F12 medium in the presence or absence of 16 $\mu$M CP32 or 24 $\mu$M CP20 (i.e., at the respective $EC_{50}$ concentrations of each of the two peptides) and their viability was measured after 24 and 48 hours. No significant differences were observed between control and treated samples, indicating that both CP32 and CP20 were not toxic to human cells (Figure 12).

Example 6: *Evaluation of CP and LP stability and design of peptidomimetic molecules of CP20 and Ac-LP20*

The evaluation of the stability of CP20 and Ac-LP20 towards proteolysis was tested at a peptide concentration of 200 $\mu$M using pronase (commercially available mixture of proteases isolated from the extracellular fluid of Streptomyces griseus, 2.5 $\mu$g/mg). Another peptide was added to this analysis, namely LP20 (without N-terminal acetylation), as it is easier to synthesize and to also assess the influence of N-terminal acetylation on the stability of LP20. Each peptide was tested in the presence of the protease (5 $\mu$g/mL) at 37°C in a buffer solution (50 mM Tris, 10 mM CaCl2, 150 mM NaCl, 0.05 % (w/v) Brij 35, pH 7.5) and its stability monitored by liquid chromatography coupled to mass spectrometry (LC-MS) at different time intervals (t=0 min, 10 min, 30 min, 1 h, 2 h). To stop enzyme activity, a small amount of the solution (60 $\mu$L) was transferred to an HPLC vial containing 6 $\mu$L of a 10% TFA solution at these different time points.

Low stability was observed for the linear peptides Ac-LP20 and LP20, which showed total proteolysis of the peptide at 10 min in the presence of protease (Figure 13A, B). Furthermore, the cut between Met and Leu was immediately observed (t0, $\beta$A-RWM residue). After 10 min in the presence of protease, in both linear peptides, proteolytic cuts between Arg 2 and Trp (residues WM and W) and between Trp and Met (residue $\beta$A-RW) were observed. For LP20, more cuts were noted than for LP20, one was observed after 10 minutes between Arg 8 and Arg 9 (residue MLVYR) and the second proteolytic cut was observed at the C-terminus of Val after 2 hours (residue MLV). Acetylation of the LP20 peptide showed no stability advantage against proteolysis compared to the unacetylated peptide (LP20). In contrast, the cyclic peptide CP20 showed good stability against proteolysis, with the cleavage between Met and Leu and between Arg 8 and Arg 9 (residue LVYR) being observed after 2 hours in the presence of protease (Figure 13C). For the same purpose, the same analyses were conducted on CP32, Ac-LP32 and LP32. A low stability of the linear peptides Ac-LP32 and LP32 was observed, as demonstrated by the total proteolysis of the peptide at 10 min in the presence of proteases (Figure 14A). For unacetylated LP32, it was immediately observed the cleavage between Trp and Leu 7 (Figure 14B, $\beta$A-GRLRW residue) after mixing protease with peptide solution in a buffer. The same cleavage was observed 10 minutes later for the acetylated peptide Ac-LP32. For both linear peptides, after 10 min in the presence of protease, cuts between Leu 4 and Arg 5 (residue RW), between Arg 5 and Trp (residues WLR, WL and W), between Leu 7 and Arg 8 (residue WL), and between Arg 8 and Val (residue WLR) were observed. The cyclic peptide CP32 is more stable against proteolysis than its linear analogues, being still observed at 10 min, but the peptide was completely cleaved at 30 min (Figure 14C). After mixing protease with peptide solution in a buffer, the GRLRWL residue showing two cleavage sites, between $\beta$Ala and Gly, and between Leu 7 and Arg 8 was observed. In addition, cleavage between Arg 8 and Val (residue V-$\beta$A-GRLRWLR) was observed.

After 10 minutes in the presence of protease, cleavages between Leu 4 and Arg 5 (residue RW), Arg 5 and Trp (residue W) and between Trp and Leu 7 (residue RV-$\beta$A-GRLRW) were observed.

From the stability-to-proteolysis results of CP20 and its linear analogue Ac-LP20, certain modifications were planned with the aim of increasing the peptide's resistance to proteolysis while maintaining good antimicrobial activity. To generate these peptidomimetic versions, LP20 was preferred as it has comparable, if not better levels of stability than Ac-LP20, as well as allowing easier synthesis by avoiding cyclization or acetylation.

The first choice was to replace leucine with aza-Leu (aL) or hydrazino-Leu (hL) to avoid possible proteolytic cuts between Met (M) and Leu (L) in Ac-LP20, LP20 and CP20 (Table 9, LR36, LR109 and LR193). Aza-amino acids are analogues of natural amino acids in which the $\alpha$-CH(R) of the amino acid residue is replaced by a nitrogen atom. They enable the peptide analogue to maintain the selectivity of natural peptides by preserving the side chain. The introduction of an aza-amino acid into peptides, in order to provide so-called aza-peptides, has shown significant success in providing biologically active peptides and increasing stability towards proteolysis [26, 27]. Hydrazino-amino acids [28] are an extension of natural amino acids in which a nitrogen atom has been added between $\alpha$-CH(R) and the amine group. They enable the peptide analogue to retain the asymmetrical $\alpha$-CH(R) carbon configuration of the natural amino acids [29].

To avoid oxidation of Met (M) in the long term (as under storage conditions), replacement of Met with its fluorinated analogue CF3-Methionine (TFM or $M_{CF3}$) in the linear and cyclic peptide LP20 (Table 9, LR131) was chosen. Furthermore, methionine is frequently involved in hydrophobic interactions and in increasing the hydrophobicity of fluorinated peptides by inducing interaction strengthening and membrane permeability [30]. D-amino acids are known to increase resistance to proteolytic degradation of the peptide due to the change in the configuration of the stereogenic alpha carbon [31].

Thus, the most significant L-amino acids in the stability test results were replaced in order to stabilise the linear peptide LP20 (Table 9, LR202 and LR206).

In particular, the following four amino acids were selected:

- Arg 2 to avoid cleavage between Arg 2 (R) and Trp 3 (W);
- Trp 3 to avoid cleavage between Trp 3 (W) and Met 4 (M);
- Tyr 7 to avoid splitting between Tyr 7 (Y) and Arg 8 (R);
- Arg 9 to avoid splitting between Arg 8 (R) and Arg 9 (R)

**Table 9.** Sequences of CP20 and its analogs generated with peptidomimetics. The results of antimicrobial tests done on the mycelium of *P. infestans* by applying the different peptides at 200 μM are provided in the column on the right, as PPI values. The letters shown next to the PPI values (%) indicate statistical significance.

| Peptide name | Sequence | PPI (%) ± Std. Dev. |
|---|---|---|
| CP20 | βA-RWMLVYRR (SEQ ID NO:1) | 92.39 ± 2.17 (a) |
| LR36 | $H_2N$-βA-RWM-**aL**-VYRR-$CONH_2$(SEQ ID NO:7) | 68.81 ± 7.03 (c) |
| LR109 (cyclic) | HN-βA-RWM-**aL**-VYRR-CO (SEQ ID NO:7) | 70.64 ± 6.7 (c) |
| LR193 | $H_2N$-βA-RWM-**hL**-VYRR-$CONH_2$ (SEQ ID NO:8) | 79.35 ± 5.47 (b) |
| LR131 | $H_2N$-βA-RW-**$M_{CF3}$**-LVYRR-$CONH_2$ (SEQ ID NO:9) | 85.32 ± 5.19 (b) |
| LR202 | $H_2N$-βA-**(D)R**-**(D)W**-MLV-**(D)Y**-R-**(D)R**-$CONH_2$ (SEQ ID NO:10) | 78.26 ± 0 (b) |
| LR206 | $H_2N$-βA-**(D)R**-**(D)W**-MLV-**(D)Y**-RR-$CONH_2$ (SEQ ID NO:11) | 81.52 ± 5.47 (b) |

The synthesis of peptidomimetic molecules was carried out using solid-phase synthesis. For the synthesis of linear peptides, a Rink amide resin was chosen to obtain a C-terminal amide. The synthesis of cyclic peptides was carried out using a 2-chlorotrimethylchloride (CTC) resin to obtain a C-terminal free carboxylic acid to directly perform head-tail cyclization.

The synthesis of non-natural amino acids (aza-Leu and hydrazino-Leu) was performed in solution before solid-phase synthesis was used [32]. For the coupling of natural amino acids, D-amino acids and CF3-Methionine, the classical coupling agents, DIC (diisopropylcarbodiimide), Oxyma and collidine, in DMF were used (HCTU/NMM or HBTU/HOBt/-DIPEA gave similar results).

For the synthesis of aza-peptides, the coupling of benzyl ester-protected natural amino acids with Fmoc-protected aza-amino acid precursors (Fmoc-2-alkyl-hydrazine) was performed using BTC (triphosgene) and DIPEA in DCM. The synthesis of these aza-peptides started with the activation of the natural amino acids with BTC followed by the addition of the aza-amino acids. Then deprotection of the acid group was performed using TriEthylSilane (TES) in MeOH, in the presence of Pd/C.

For the synthesis of hydrazino-peptides, hydrazino-amino acid coupling was performed using DEPBT (3-(diethoxyphosphoryloxy)-1,2,3-benzotriazin-4(3H)-one) and DIPEA in DMF.

Example 7: *Evaluation of the antimicrobial activity of peptidomimetic molecules CP20 and LP20*

In order to assess the efficacy of the peptidomimetics described above, an assay was performed at 200 μM against *P. infestans* mycelium in the manner described above. Despite the substitution of some main sequence components with non-proteinogenic amino acids, the peptidomimetics tested showed good abilities in limiting mycelial growth. Indeed, the seven peptides showed relatively high PPI values, with growth inhibitions close to or above 70% (Table 9). In general, it can be said that some peptidomimetic versions (LR131 and LR206) presented PPIs between 80 and 85%, thus also presenting themselves as improved over LP20 on solid medium (72.78 % ± 5.41 %). This observation indicates that these peptides, although linear, may present a good framework for introducing modifications to improve their stability and achieve good antimicrobial activities.

## REFERENCES

[1] Thines, 2018, Current Biology. 28: R812-R813
[2] Fisher et al., 2012, Nature. 484: 186-194
[3] Haverkort et al., 2008, Potato Research. 51: 47-57
[4] Sun et al., 2008, Can. J. Plant Pathol. 30: 414-424
[5] Erwin and Ribeiro, 1996, Phytophtora diseases worldwide; ISBN : 9780890542125
[6] Alhussaen, 2012. J. Biol. Sci. 12: 416-420
[7] Gessler et al., 2011. Phytopathologia Mediterranea. 50: 3-44
[8] Agrios, 2005. Plant Pathol. 26-27: 398-401
[9 Komarek et al., 2010. Environment International. 36: 138-151
[10] Wightwick et al., 2008. J. Agric. Food Chem. 56: 2457-2464
[11] Corsini et al. 2013. Toxicology. 307: 123-135

[12] Polyxeni et al. 2016. Front. Public Health. 4: 148

[13] Rosa et al., 2023. Front. Mol. Biosci. 10: 1017757

[14] Grenville-Briggs et al., 2008. Plant Cell. 20: 720-738

[15] Colombo et al., 2020. Scientific. Reports. 10: 17574

[16] Blum et al., 2010. Fungal Genet. Biol. 47: 499-510

[17] Barreto and Geyer, 2014. Yeast Protocols. 4562: 273-309

[18] Barreto et al., 2009. Chemistry&Biology. 16: 1148-1157

[19] Barreto et al., 2009. Chemistry&Biology. 16: 1148-1157

[20] Pellegrino et al., 2012. Amino Acids. 43: 1995-2003

[21] Grenville-Briggs et al., 2008. Plant Cell. 20: 720-738

[22] Blum et al., 2010. Fungal Genet. Biol. 47: 499-510

[23] Colombo et al., 2020. Sci. Rep. 10: 17574

[24] Fugelstad et al. 2009. Fungal Genet. Biol. 46: 759-767

[25] Colombo et al., 2020. Sci. Rep. 10: 17574

[26] Proux et al., 2011. Chem Biol Drug Des. 78: 887

[27] Begun et al., 2017. Asian J Chem. 29: 1879

[28] Katoh and Suga 2021. J. Am. Chem. Soc. 143, 45: 18844-18848

[29] Guy et al., 1998. J. Med. Chem. 41, 24: 4833-4843

[30] Gadais et al., 2018. Chem Bio Chem. 19: 1026

[31] Hamamoto et al., 2002. Microbiol. Immunol. 46: 741

[32] Chingle et al., 2014. Org. Lett. 16: 5434

[33] Ritz, C. et al. (2015) Dose-Response Analysis Using R. PLoS One 10, e0146021.

## Claims

1. Linear or cyclic peptide consisting of a β-amino acid and 8 L-amino acids having the following sequence (I):

$$X - R - (Y_1 - Y_2 - Y_3 - Y_4 - Y_5 - Y_6) - Z \qquad (I)$$

wherein X is selected among the group consisting of β-Ala (βA), Ala (A), Gly (G), gamma amino butyric acid or a derivative thereof;

R is an L-amino acid selected between Arg (R) or Gly (G);

$Y_1$, $Y_2$, $Y_3$, $Y_4$, $Y_5$ and $Y_6$ are L-amino acid selected from the group consisting of Trp (W), Leu (L), Met (M), Tyr (Y), Arg (R), Ala (A), Gly (G) or Val (V);

Z is an L-amino acid selected between Arg (R) or Val (V);

with the proviso that the amino acid Arg (R) is present at least three times and Trp (W), Met (M), Tyr (Y) and Val (V) are present once in the sequence of formula (I), or their peptidomimetic variants,

as antimicrobial or fungicide agent in agronomic field.

2. Linear or cyclic peptide according to claim 1, wherein the derivative of β-Ala (βA), Ala (A), Gly (G) or gamma amino butyric acid is the amino acid which is substituted on alpha, beta or gamma position by an alkylCO group, wherein alkyl is a linear or branched alkyl group $C_1$-$C_4$.

3. Linear or cyclic peptide according to claim 2, wherein the derivative of β-Ala is acetyl-βA (Ac-βA).

4. Linear or cyclic peptide according to anyone of claims 1-3, wherein:

   $Y_1$ is Trp (W) or Arg (R);
   $Y_2$ is Met (M) or Leu (L);
   $Y_3$ is Leu (L) or Arg (R);
   Y4 is Val (V) or Trp (W);
   $Y_5$ is Tyr (Y) or Leu (L) and
   $Y_6$ is Arg.

5. Linear or cyclic peptide according to anyone of claims 1-4, selected from the group consisting of:

βA-R-W-M-L-V-Y-R-R (SEQ ID NO:1)
βA-G-R-L-R-W-L-R-V (SEQ ID NO:2)
Ac-βA-R-W-M-L-V- Y - R - R-CONH$_2$ (SEQ ID NO:3)
Ac-βAla - Gly - Arg - Leu - Arg - Trp - Leu - Arg - Val - CONH$_2$ (SEQ ID NO:4) or their peptidomimetic variants.

6. Linear or cyclic peptide according to anyone of claims 1-5, wherein said peptidomimetic variants derive from the incorporation of not proteinogenic amino acids, such as D-amino acids, N-alkyl glycines, fluorinated amino acids, aza amino acids, hydrazino acids or beta-amino acids.

7. Linear or cyclic peptide according to claim 5, wherein said peptidomimetic variant is selected from the group comprising the following sequences:

βA-R-W-M-aL-V-Y-R-R (SEQ ID NO:7)
βA-R-W-M-hL-V-Y-R-R (SEQ ID NO:8)
βA-R-W-M$_{CF3}$-L-V-Y-R-R (SEQ ID NO:9)
βA-(D)R-(D)W-M-L-V-(D)Y-R-(D)R (SEQ ID NO:10)
βA-(D)R-(D)W-M-L-V-(D)Y-R-R (SEQ ID NO:11)
βA-R-(D)W-M-L-V-(D)Y-R-R (SEQ ID NO:12)
βA-R-W-A-hL-V-Y-R-R (SEQ ID NO:13)
βA-R-(D)W-M-L-V-(D)Y-R-R (SEQ ID NO: 14)
βA-G-R-L-(D)R-(D)W-L-R-V (SEQ ID NO:15)
βA-GRL(D)R-W-D(L)-R-V (SEQ ID NO:16)
βA-G-(D)R-L-(D)R-(D)W-L-R-V (SEQ ID NO:17)
βA-G-R-L-R-(D)W-(D)L-R-V (SEQ ID NO:18)
βA-G-R-L-R-(D)W-L-(D)R-V (SEQ ID NO:19)
βA-G-(D)R-L-R-(D)W-L-R-V (SEQ ID NO:20)
βA-G-R-L-R-ahomoW-L-R-V (SEQ ID NO:21)
βA-G-R-L-R-W-aL-R-V (SEQ ID NO:22)
βA-G-R-L-R-hW-L-R-V (SEQ ID NO:23)
βA-G-R-L-R-W-hL-R-V (SEQ ID NO:24)
βA-G-R-hL-R-W-L-R-V (SEQ ID NO:25)
wherein aL is aza-Leu; ahomoW is aza-homoTrp, hL is hydrazino-Leu; hW is hydrazino-Trp; MCF3 is CF3-Met and (D) indicates that it is a D-amino acid instead of L-amino acid.

8. Use of the linear or cyclic peptide according to any one of the claims 1-7, as antimicrobial or fungicide agent in agronomic field.

9. Use of the linear or cyclic peptide according to anyone of claims 1-7, for treating or preventing the infections mediated by Oomycetes.

10. Use of the linear or cyclic peptide according to claim 9, wherein said Oomycetes are selected from the taxonomic families of *Peronosporales* and *Pythiaceae.*

11. Use of the linear or cyclic peptide according to claim 9 or 10, for treating or preventing *Phytophthora infestans, Phytophthora capsici, Plasmopara viticola, Peronospora spp, Phytophtora spp* and *Phythium ultimum* infection.

12. Phytopharmaceutical composition comprising at least one linear or cyclic peptide according to any one of the claims 1-7 as active principle, and optionally a solid or liquid solvent and /or diluent, at least an adjuvant and/or co-formulating agent.

13. Phytopharmaceutical composition according to claim 12, comprising one or more further active ingredients such as a fungicide other than the linear or cyclic peptide according to any one of the claims 1-7, selected from the groups consisting of phytoregulators, antibiotics, herbicides, insecticides, fertilizers and/or mixtures thereof.

14. Method for controlling phytopathogenic fungi in agricultural crops, which consists in applying, on any part of the plants to be protected or on the ground, effective and non-phytotoxic doses of a linear or cyclic peptide according to any of the claims 1-7, used as such or formulated in phytopharmaceutical composition according to anyone of claims 12-13.

15. Method for controlling phytopathogenic fungi according to claim 14, wherein said phytopathogenic fungi belong to Oomycetes class and are selected from the group consisting of *Phytophthora infestans, Phytophthora capsici, Plasmopara viticola, Peronospora spp, Phytophtora spp* and *Phythium ultimum.*

16. Method for controlling phytopathogenic fungi according to any one of the claims 14-15, wherein said agricultural crops comprising fruit bearing crops, preferably vine, tomato, potato, maize, wheat and horticultural crops.

17. A nucleotide sequence encoding for one of the linear or cyclic peptide of formula (I) according to anyone of claim 1-7.

18. A genetic construct comprising at least one nucleotide sequence according to claim 17.

19. A transgenic plant expressing at least one nucleotide sequence encoding for a linear or cyclic peptide according to anyone of claim 1-7.

### *Phytophthora infestans* Cellulose synthase 3: protein sequence

MGLTGAGIIASVVGILGGVSLSCGGWSSLSLGARSLFVTTQFLSAFAMGFVVAFSAIVSLSDTNEW
VAVAAGGGAGFVIALIGGFMTIFGPYILILITGGLIACYLLLVDAYDGINVFPADNQLARQEFVIA
FMIIFELVCCSSSKTSELENHRFKYIVFSAITGGWMAADGVSRLIDSGAVLSTVAYTSIQDGGKAA
MDGIDASAQTLMFVIWGAVFVVGGLNQLSMRWGLMCYNRVGAHAQLGPVEEQMPELPTGATLPAQT
MTERVRLVCENCFATVPAGTAFCTECGEAMPSEDGNPDVSISQAQMPSVSMNNKGQVPDRWQHVPH
RTYMSTTSFVDPKHAKEGGVSMKDNGRSIRFMDSGVQGPDGKMSQYNDSIAGVRNYYEPSFRSFAM
STYSIANRAAEPVETPNIRKYKMSGSGMFHVFYFGTAATGIFWLYYLTTMYPQQYFCDHARPTLPC
SGLPTSETTGCYSSTVNFDADSGDGYCIKDVPFMSWLMYAMMIFSEFLNYFLGLLFNFSMW**RPIRR**
**GARYMNDFKPPIPKEQWPTVDIFLCHYMEPVTDSMQTLKNCLAMQYPPELLHIFILDDGYTKSVWD**
**ANNHFKVTVNTKVIEVAGDLRGDLARLMHERVVGPVQDDQSLKSWRRQHSSVRELRKEGGKGVQRR**
**DCAVGSLSDDYDYRDRGIPRVTFIGRMKPETHHSKAGNINNALFNEGADGKYLLILDNDMKPHPKF**
**LLAVLPFFFSEGEAVDGGGRQYSDDISWNQVSYVQTPQYFEDTPQLTIMGDPCGHKNTIFFDAVQC**
**GRDGFDSAAFAGTNAVFRRQAFDSIGGICYGTQTEDAYTGNVLHTSGWDSVYFRKDFEGDAKDRIR**
**LCEGAVPDTVAAAMGQKKRWAKGAVQILLMKNESEVDPDWRPPRVPAPDPKPSLTFPRKMFFYDSV**
**LYPLG**SIPALCYVSIAVYYLCTGDAPIYARGTKFLYSFLPVTFCRWVLNLLANRAVDNNDVWRAQQ
TWFSFSFITMMAIVEAIQARVTGKDKSWANTGAGQKTSWTEIPNVLFFFTLLFSQLVALVRFFEYE
NATNPWNYVSAMFFGFFVMSQFYPMVKMSITEYCGWDHTAATFTANVFGSLLVVYIVVFVQLWQVY
YEGNLQVAQGTDAGGSAAAT*

### *Phytophthora infestans* Cellulose synthase 2: protein sequence

MYGNDKQSLMKHEDYELHGTPATGANDGGAGFYAQEGRPTPQQGYVDPRGPALPPMNVSDAVGLGG
QRDNIISVHGYMHKQGKRTIKGPIHKSWKRRYFALEKAKIYYFYSHLECRQYFTTRNADLVVGAIE
LKDALQLRPCARLDLPHKGFEVHTKRRVWVLCPETDEEYRMWFQGVERAIVANGAGNIIERKLPNV
RKYLMKGNQTYRFFYFLFLIAGIVELLAIVFWFVIGLEPCDASRLEVDCATITSTSLETLRCSNEP
FSGWFTPPDWYLEVADVDNVICFRDPPIPQWASYFAMLFAEVLTFALGVLYYLGMWKPVRRGAHYF
DEFEPPVPDDLWPKVDVLLCHYSEPAEETIDTLMACMNLQYPPHLLQIWVCDDGYCKTKWTKGNPV
PTVELNKGILETAGDLRQEVAQFMYDRVCDPNEDMEVYAWRKLHSSANLPSPSRVKVVNRADCAVG
SFRDDYRYPGLPHVTFIGRVKPETHYSKAGNINNCLYNEGANGRYLIILDTDMQPHPKFILATLPF
FFDDEDRQDKAKYICCGIGCNAVAKLCCASCQIAGVPEEQISYCSKDCFENAMHVQSAVHRRQVNG
TMSDTRASKIDMRCMNCDAKLPKNGVCRKCGNHGADGEDVSSLHNYSDD**VRDNAVAFVQTPQYFRD**
**CIQLQIGDPMGHRNATFYDAIQTGQDGYDCASFAGTNAMFRREALDSIGGIQYGSLTEDCYTGQVL**
**CSMGWKAQYFRKDFEGEPSERIRLAEGLIPDSVAGSLAQRKRWAKGNFQIAL**MNKKTQYFDPEWKM
PEAQIPSYHKSNKFMRRVFYFNSTLYPLGSITAILFYYITLYFLFSGYAPIYMAGARLVYALVPKL
LIQGVLSALSNRTVDNSDVIRSQEVWFAYAFTNCTAVLEAFWWKITGKEPKWFNTGGASRGSTAEL
PNVIIFFGTVVGVLWSVVRFLAGYNSIQTSHGASLLFASLMMGLFIAVKLAPSVRMSIQEYFGWSY
ESLMDQGNVVGSISIAFGLIFITLWVWIEQPTSNPF*

**FIG. 1**

FIG. 2

FIG. 3

| Not-Treated | CP20 – 200 µM | CP32 – 200 µM | Mandipropamid 10 mg/l |

FIG. 4

**A**

**CP20**

$$f(x) = \frac{99.96}{1 + \exp(-0.10(x - 23.75))}$$

CP20 (µM)

**CP32**

$$f(x) = \frac{98.14}{1 + \exp(-0.12(x - 16.34))}$$

CP32 (µM)

**B**

| ECx | Dose (µM) | Errore std. | IC 95% |
|---|---|---|---|
| 50 | 23.75 | 2.99 | 17.44 − 30.05 |
| 99 | 68.37 | 12.71 | 41.54 − 95.20 |

| ECx | Dose (µM) | Errore std. | IC 95% |
|---|---|---|---|
| 50 | 16.34 | 2.35 | 11.55 − 23.14 |
| 99 | 56.18 | 8.54 | 38.80 − 73.56 |

**FIG. 5**

**A**

Not-Treated

Mandipropamid (10 mg/l)

CP20 (200 µM)

CP32 (200 µM)

**B**

| CP20 | CP32 | Mandipropamid |
|---|---|---|
| 88.64 ± 2.12 | 90.03 ± 0.85 | 100 ± 0 |

**FIG. 6**

Not-Treated

Mandipropamid
(10 mg/l)

CP20 (200 μM)

CP32 (200 μM)

FIG. 7

**FIG. 8**

**FIG. 9**

**FIG. 10**

**FIG. 11**

**HKC (Human Kidney Cell)**

FIG. 12

FIG. 13

FIG. 14

**EP 4 549 575 A1**

Europäisches Patentamt
European Patent Office
Office européen des brevets

Application Number
**EP 23 20 7559**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | XI XUEDONG ET AL: "Two novel cyclic depsipeptides Xenematides F and G from the entomopathogenic bacteriumXenorhabdus budapestensis", THE JOURNAL OF ANTIBIOTICS, NATURE PUBLISHING GROUP UK, LONDON, vol. 72, no. 10, 2 July 2019 (2019-07-02), pages 736-743, XP036881197, ISSN: 0021-8820, DOI: 10.1038/S41429-019-0203-Y [retrieved on 2019-07-02] * page 736, column 1, line 1 - column 2, line 8 * * figure 1 * | 1-19 | INV. C12N15/82 A01N63/50 |
| Y | US 2008/032924 A1 (EAST PETER DAVID [AU] ET AL) 7 February 2008 (2008-02-07) * claims 1,14 * * paragraphs [0270], [0217], [0210] * | 1-19 | |
| Y,D | COLOMBO MONICA ET AL: "NoPv1: a synthetic antimicrobial peptide aptamer targeting the causal agents of grapevine downy mildew and potato late blight", SCIENTIFIC REPORTS, vol. 10, no. 1, 16 October 2020 (2020-10-16), XP93142760, US ISSN: 2045-2322, DOI: 10.1038/s41598-020-73027-x Retrieved from the Internet: URL:https://www.nature.com/articles/s41598-020-73027-x> * abstract; figure 2 * | 1-19 | TECHNICAL FIELDS SEARCHED (IPC) C12N A01N |

-/--

The present search report has been drawn up for all claims

| Place of search | Date of completion of the search | Examiner |
|---|---|---|
| Munich | 25 March 2024 | Obel, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Rosa: "CHARACTERIZATION OF BIOACTIVE PEPTIDES IDENTIFIED FROM GENETICALLY ENCODED LIBRARIES.",<br>,<br>1 January 2023 (2023-01-01), XP093142788,<br>Retrieved from the Internet:<br>URL:https://hdl.handle.net/2434/981728<br>* the whole document * | | |

TECHNICAL FIELDS
SEARCHED       (IPC)

The present search report has been drawn up for all claims

| Place of search | Date of completion of the search | Examiner |
|---|---|---|
| Munich | 25 March 2024 | Obel, Nicolai |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**Application Number**

**EP 23 20 7559**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

The Search Division considers that the present European patent application does not comply with the requirements of unity of invention and relates to several inventions or groups of inventions, namely:

**1. claims: 1-19**

**Peptides for controlling plant pests.**

**1.1. claims: 1-19(partially)**

**Directed to peptide having SEQ ID NO 1.**

**1.2. claims: 1-19(partially)**

**Each further peptide as defined in claims 5 and 6 represent an independent invention.**

---

Please note that all inventions mentioned under item 1, although not necessarily linked by a common inventive concept, could be searched without effort justifying an additional fee.

## ANNEX TO THE EUROPEAN SEARCH REPORT
## ON EUROPEAN PATENT APPLICATION NO.

**EP 23 20 7559**

This annex lists the patent family members relating to the patent documents cited in the above-mentioned European search report.
The members are as contained in the European Patent Office EDP file on
The European Patent Office is in no way liable for these particulars which are merely given for the purpose of information.

**25-03-2024**

| Patent document cited in search report | Publication date | Patent family member(s) | | Publication date |
|---|---|---|---|---|
| US 2008032924 A1 | 07-02-2008 | CA | 2557333 A1 | 01-09-2005 |
| | | CN | 1950396 A | 18-04-2007 |
| | | EA | 200601533 A1 | 31-08-2007 |
| | | EP | 1730180 A1 | 13-12-2006 |
| | | US | 2008032924 A1 | 07-02-2008 |
| | | WO | 2005080423 A1 | 01-09-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **THINES**. *Current Biology*, 2018, vol. 28, R812-R813
- **FISHER et al.** *Nature*, 2012, vol. 484, 186-194
- **HAVERKORT et al.** *Potato Research*, 2008, vol. 51, 47-57
- **SUN et al.** *Can. J. Plant Pathol.*, 2008, vol. 30, 414-424
- **ERWIN** ; **RIBEIRO**. *Phytophtora diseases worldwide*, 1996, ISBN 9780890542125
- **ALHUSSAEN**. *J. Biol. Sci.*, 2012, vol. 12, 416-420
- **GESSLER et al.** *Phytopathologia Mediterranea*, 2011, vol. 50, 3-44
- **AGRIOS**. *Plant Pathol.*, 2005, vol. 26-27, 398-401
- **KOMAREK et al.** *Environment International.*, 2010, vol. 36, 138-151
- **WIGHTWICK et al.** *J. Agric. Food Chem.*, 2008, vol. 56, 2457-2464
- **CORSINI et al.** *Toxicology*, 2013, vol. 307, 123-135
- **POLYXENI et al.** *Front. Public Health*, 2016, vol. 4, 148
- **ROSA et al.** *Front. Mol. Biosci.*, 2023, vol. 10, 1017757
- **GRENVILLE-BRIGGS et al.** *Plant Cell*, 2008, vol. 20, 720-738
- **COLOMBO et al.** *Scientific. Reports.*, 2020, vol. 10, 17574
- **BLUM et al.** *Fungal Genet. Biol.*, 2010, vol. 47, 499-510
- **BARRETO** ; **GEYER**. *Yeast Protocols*, 2014, vol. 4562, 273-309
- **BARRETO et al.** *Chemistry&Biology*, 2009, vol. 16, 1148-1157
- **PELLEGRINO et al.** *Amino Acids.*, 2012, vol. 43, 1995-2003
- **COLOMBO et al.** *Sci. Rep.*, 2020, vol. 10, 17574
- **FUGELSTAD et al.** *Fungal Genet. Biol.*, 2009, vol. 46, 759-767
- **PROUX et al.** *Chem Biol Drug Des.*, 2011, vol. 78, 887
- **BEGUN et al.** *Asian J Chem.*, 2017, vol. 29, 1879
- **KATOH** ; **SUGA**. *J. Am. Chem. Soc.*, 2021, vol. 143 (45), 18844-18848
- **GUY et al.** *J. Med. Chem.*, 1998, vol. 41 (24), 4833-4843
- **GADAIS et al.** *Chem Bio Chem.*, 2018, vol. 19, 1026
- **HAMAMOTO et al.** *Microbiol. Immunol.*, 2002, vol. 46, 741
- **CHINGLE et al.** *Org. Lett.*, 2014, vol. 16, 5434
- **RITZ, C. et al.** Dose-Response Analysis Using R. *PLoS One*, 2015, vol. 10, e0146021